(19) Europäisches Patentamt
European Patent Office
Office européen des brevets

(11) EP 0 685 444 B1

(12) FASCICULE DE BREVET EUROPEEN

(45) Date de publication et mention de la délivrance du brevet:
**05.04.2000 Bulletin 2000/14**

(21) Numéro de dépôt: **95107094.5**

(22) Date de dépôt: **10.05.1995**

(51) Int Cl.[7]: **C07C 47/228**, C07C 47/235, C07C 47/453, C07D 317/12, C07C 69/007, C07C 33/34, C11B 9/00, A61K 7/46, C07C 45/54, C07C 45/62, C07C 45/30, C07C 45/68

# (54) Composés aromatiques nouveaux et leur utilisation en parfumerie

Aromatische Verbindungen und ihre Verwendung in der Parfümerie

Aromatic compounds and their use in perfumery

(84) Etats contractants désignés:
**CH DE ES FR GB IT LI NL**

(30) Priorité: **31.05.1994 CH 168994**

(43) Date de publication de la demande:
**06.12.1995 Bulletin 1995/49**

(73) Titulaire: **FIRMENICH SA**
**1211 Genève 8 (CH)**

(72) Inventeurs:
- **Winter, Beat**
  **CH-1233 Bernex (CH)**
- **Blanc, Pierre-Alain**
  **CH-1263 Crassier (CH)**
- **Lamboley, Serge**
  **CH-1030 Bussigny (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**WO-A-94/27946** **FR-A- 1 177 132**
**GB-A- 2 079 751**

- **PATENT ABSTRACTS OF JAPAN vol. 014 no. 555 (C-0786) ,10 Décembre 1990 & JP-A-02 238097 (KURARAY CO LTD) 20 Septembre 1990,**

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).

EP 0 685 444 B1

Printed by Jouve, 75001 PARIS (FR)

## Description

La présente invention a trait au domaine de la parfumerie. Elle concerne, en particulier, des composés aromatiques nouveaux, utiles à titre d'ingrédients parfumants, de formule

a)

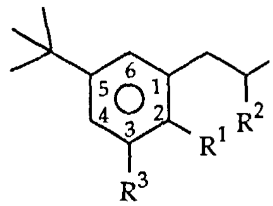

(Ia)

dans laquelle le symbole X représente un groupe - CHO ou un groupe

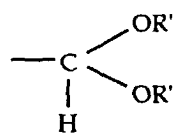

les symboles R', pris isolément, représentant chacun un radical alkyle de $C_1$ à $C_4$, linéaire ou ramifié, saturé ou insaturé, ou, pris ensemble, représentant un radical alkylène de $C_2$ à $C_4$, éventuellement substitué ; le symbole $R^2$ représente un atome d'hydrogène ou un radical méthyle ; et $R^1$ et $R^3$ sont différents et représentent chacun un atome d'hydrogène ou un radical méthyle, le 2-(5-tert-butyl-2-méthylbenzyl)propanal étant exclu ;
ou de formule
b)

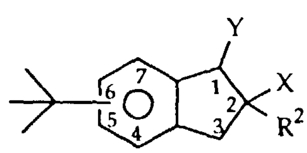

(Ib)

dans laquelle le radical tert-butyle se trouve en position 5 ou 6 du cycle aromatique et soit Y représente l'hydrogène et X et $R^2$ ont le sens indiqué ci-dessus, soit X et $R^2$ représentent chacun un atome d'hydrogène et Y représente un groupe - $CH_2CHO$ ou un groupe

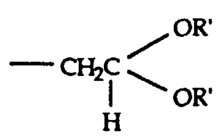

où R' est défini comme en a) ;
ou de formule
c)

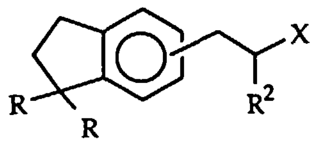

(Ic)

dans laquelle X et $R^2$ ont le sens indiqué à la formule (Ia) et R représente un atome d'hydrogène ou un radical méthyle, les groupes R pouvant être identiques ou différents.

Comme il ressort du tableau suivant, on connaît de l'art antérieur plusieurs composés de structure proche de celle des composés (Ia), dont certains ont rencontré du succès commercial. Plusieurs de ces composés sont d'ailleurs décrits dans l'oeuvre de référence de S. Arctander, Perfume and Flavor Chemicals, Montclair, N.J., USA (1969) et ceci est indiqué dans le tableau.

TABLEAU

| Composés | Odeur | Référence |
| --- | --- | --- |
| 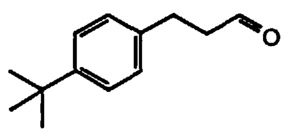 | très forte, florale type muguet | Naarden Int. - feuille de produit |
| 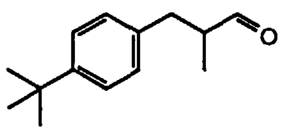 | florale, verte, fleur de tilleul | Arctander 496 |

TABLEAU (suite)

| Composés | Odeur | Référence |
|---|---|---|
| 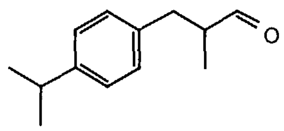 | florale, verte, concombre, melon, fleur de tilleul | Arctander 758 |
| 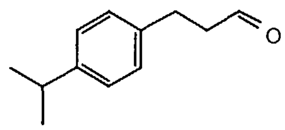 | florale, douce, verte fruitée | Arctander 2741 |
| 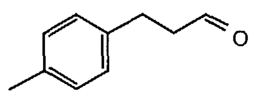 | florale, douce, épicée | Arctander 2073 |
| 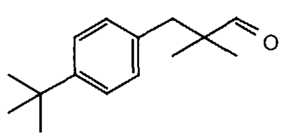 | florale, verte, muguet | NL 7905175 |
| 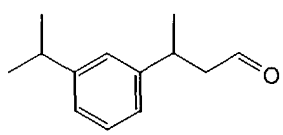 | fraîche, florale, fruitée, melon, muguet, fleur de tilleul | US 4'910'346 |

Le brevet français 1.177.132 décrit le 3-(4-tert-butylphényl)-2-méthylpropanal, mentionné dans le Tableau ci-dessus, deuxième ligne, et connu sous le nom de Lilial ® (voir ci-dessous), et son utilité en tant qu'ingrédient parfumant. Ledit brevet mentionne également d'autres aldéhydes de structure similaire, dont le 2-(5-tert-butyl-2-méthylbenzyl) propanal, dont il est dit qu'il "ne possède pas une valeur olfactive remarquable", ne les qualifiant pas en tant qu'ingrédient parfumant.

On connait également, de la demande japonaise JP 2238097, le 2-(5-tert-butylbenzyl)propanal de formule générale

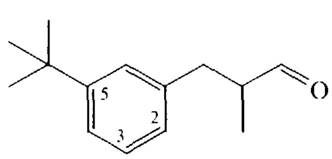

dont l'odeur est décrite comme étant du type muguet, avec un connotation verte. Cependant, ce document ne suggère nullement ni les composés décrits dans la présente demande, ni le fait que lesdits composés, étant analogues du 2-(5-tert-butylbenzyl)propanal pourraient s'avérer utiles dans le domaine de la parfumerie.

Malgré l'abondance de produits connus de ce type, l'activité de recherche dans ce domaine reste soutenue, notamment dans le but de trouver des composés présentant des nuances odorantes plus variées et aussi des composés dont la stabilité en composition soit améliorée par rapport aux produits connus. En effet, on sait que ces aldéhydes de l'art antérieur s'oxydent très facilement à l'air pour fournir les acides correspondants, lesquels sont inodores ou d'une odeur désagréable mais, en tout cas, ne possèdent plus du tout les caractères olfactifs recherchés.

Or, nous avons maintenant découvert de façon surprenante que les composés (la, b, c) possèdent des propriétés odorantes très utiles et distinctes de celles des produits de l'art antérieur, et qu'ils présentent également des avantages du point de vue de la stabilité à l'oxydation et de la tenacité de leur note odorante. Nous avons notamment constaté que certains composés préférés de l'invention pouvaient remplacer avec bonheur, dans leurs applications typiques, leurs analogues connus, à savoir le 3-(4-tert-butyl-1-phényl)-2-méthylpropanal, ou LILIAL® (origine : Givaudan-Roure, Vernier, Suisse), et le 3-(4-tert-butyl-1-phényl)propanal, ou BOURGEONAL ® (origine : Naarden Intl, Hollande), et qu'ils pouvaient se montrer beaucoup plus tenaces et stables que ces derniers lorsqu'utilisés, par exemple, dans des détergents et adoucissants textiles.

C'est ainsi que, parmi les composés de l'invention, on peut citer, à titre préférentiel, le 3-(5-tert-butyl-2-méthyl-1-phényl)propanal lequel développe une odeur florale, verte, très puissante, avec un caractère qui rappelle l'odeur du thym. Son homologue méthylé dans la chaîne, à savoir le 2-(5-tert-butyl-2-méthylbenzyl)propanal, possède lui une odeur floralemuguet très puissante et élégante, aussi d'une bonne tenacité.

Curieusement, des isomères de ces deux derniers composés de l'invention, à savoir les 3-(3-tert-butyl-5-méthylphényl)propanal et 3-(3-tert-butyl-5-méthylphényl)-2-méthylpropanal, exhalent des odeurs tout à fait distinctes, le premier possédant une note racineuse, terreuse, de type vétyver, avec un caractère de fond floral, vert, très puissant, et le second développant une odeur ozonée très appréciée.

Parmi les composés bicycliques de formules (Ib,c), on peut citer à titre préférentiel le 5-tert-butyl-2-indanecarbaldéhyde dont l'odeur est particulièrement puissante et tenace, de type floral, vert, rappelant l'odeur du BOURGEONAL®, possédant aussi une nuance aqueuse-ozonée qui le rend très intéressant. Le 5-tert-butyl-2-méthyl-2-indanecarbaldéhyde, ayant un radical méthyle de plus, développe lui une odeur tout à fait différente, à caractère métallique, aldéhydé, vert, aussi aqueux et faiblement phénolique.

Un autre composé très apprécié des parfumeurs est le 3-(3,3-diméthyl-5-indanyl)propanal, à odeur ozonée et verte, fleurie, aldéhydée, d'une puissance rare et aussi très tenace. Sa note odorante évoque instantanément l'odeur de linge propre. Tel qu'obtenu directement de la synthèse décrite plus loin, ce composé peut être accompagné d'une quantité mineure de son isomère 3-(1,1-diméthyl-5-indanyl)propanal, dont l'odeur est similaire, et les mélanges des deux composés ont un caractère olfactif du même type que celui décrit ci-dessus.

Encore un composé préféré de l'invention est le 6-tert-butyl-1-indaneacétaldéhyde, dont l'odeur ressemble à celle du 5-tert-butyl-2-indanecarbaldéhyde cité plus haut, tout en possédant une note crésylique plus prononcée et un côté plus vert-anisique et moins aldéhydé-muguet que ce dernier.

On peut citer également, à titre préférentiel, le 3-(5-indanyl)propanal dont l'odeur est de type aldéhydé, type BOURGEONAL®, avec une nuance de fond héspéridée et une puissance remarquable. Ce composé peut aussi être employé en mélange avec son isomère 3-(4-indanyl)propanal pour conférer les caractères olfactifs mentionnés.

Il ressort de ce qui précède que les composés de l'invention apportent à la palette du parfumeur une gamme nouvelle de nuances odorantes très variée, tout en conservant certains des caractères les plus appréciés dans les composés de l'art antérieur de structure analogue cités dans le Tableau.

D'autre part, et comme il ressort des exemples présentés plus loin, il a été découvert que certains composés (Ia), dont la structure est plus proche de celle des composés connus, se révèlent beaucoup plus stables contre l'oxydation que les composés de l'art antérieur et présentent des notes odorantes beaucoup plus tenaces, ce qui les rend d'un usage nettement plus avantageux dans des milieux particulièrement agressifs, tels que détergents et produits d'entretien.

Ce résultat de nos recherches était d'autant plus inattendu que, malgré le grand nombre de composés nouveaux préparés, de l'ordre d'une cinquantaine, en variant la nature et la position des substituants et la nature du groupe fonctionnel (des dérivés esters, éthers et nitriles ayant également été préparés), seuls quelques composés ont vraiment suscité l'intérêt des parfumeurs, parmi lesquels les composés (Ia, b, c) ont montré des caractères odorants exceptionnels et, notamment ceux où $R^2$ représente un atome d'hydrogène, une stabilité accrue par rapport aux composés connus. Ces propriétés avantageuses se sont révélées lors de laborieuses évaluations olfactives entreprises par des panels d'experts parfumeurs, lesquels ont découvert avec surprise la supériorité des qualités odorantes de ces composés, supériorité bien évidente non seulement lors de l'évaluation sur mouillette du produit à l'état pur ou en composition, mais aussi lors de son application pour le parfumage notamment de détergents et adoucissants textiles.

Les composés de l'invention trouvent un emploi avantageux aussi bien en parfumerie fine, qu'en parfumerie fonctionnelle et, de par leurs propriétés odorantes, leur application est bien plus étendue que celle des composés connus et, notamment de l'aldéhyde p-tert-butyl-$\alpha$-méthyl-hydrocinnamique connu. Ils servent à préparer des bases parfumantes et des parfums et sont également fort utiles pour le parfumage d'articles de consommation divers tels les savons, les gels de douche ou bain, les shampoings et produits après-shampoing, les préparations cosmétiques et les désodorisants corporels ou d'air ambiant. D'autre part, grâce à la puissance et à la substantivité de leur note, les

5-tert-butyl-2-indanecarbaldéhyde et 3-(3,3-diméthyl-5-indanyl)propanal en particulier se sont révélés d'un emploi très avantageux dans le parfumage de détergents ou adoucissants textiles. Les produits d'entretien peuvent également être parfumés à l'aide des composés (Ia, b, c).

Dans ces applications, on peut les utiliser dans une gamme de concentrations fort étendue. A titre d'exemple on peut citer des concentrations de l'ordre de 5 à 10%, voire même 15 ou 20% en poids, par rapport au poids de la composition dans laquelle ils sont incorporés.

Il est clair cependant que ces valeurs ne peuvent être données qu'à titre indicatif, puisque les concentrations de composé (I) dépendent, et de l'effet olfactif désiré, et de la nature du produit que l'on désire parfumer. D'autre part, elles sont également dépendantes de la nature des autres ingrédients dans une composition donnée, lorsque les composés (I) sont utilisés en mélange avec des solvants, adjuvants et co-ingrédients parfumants d'usage courant, dont on peut trouver des exemples dans des ouvrages de référence tels que le livre de S. Arctander, Perfume & Flavor Chemicals, Montclair, N. J., USA (1969).

Des valeurs bien inférieures à celles citées plus haut, de l'ordre de 0,1 à 0,5% en poids, par rapport au poids de la composition dans laquelle ils sont incorporés, seront normalement utilisées lors de l'emploi des composes (I) dans le parfumage des articles de consommation divers susmentionnés.

L'invention concerne également un procédé de préparation d'un composé de formule

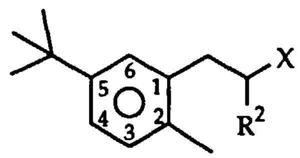

(I'a)

dans laquelle les symboles X et $R^2$ ont le sens indiqué à la formule (Ia), ou de formule

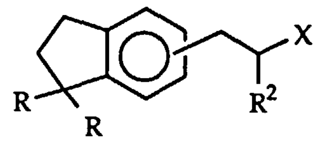

(Ic)

telle que définie précédemment, le procédé étant caractérisé en ce qu'on hydrolyse, à l'aide d'un acide, un énol-ester de formule

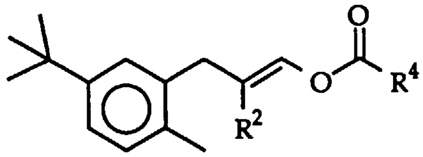

(IIa)

ou, respectivement, de formule

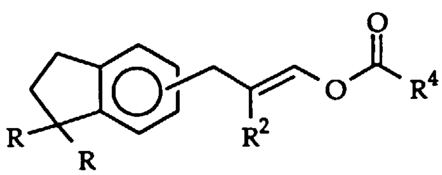

(IIc)

dans laquelle les symboles R et $R^2$ ont le sens indiqué ci-dessus et le symbole $R^4$ représente un radical alkyle de $C_1$ à $C_3$ et, le cas échéant, on acétalyse, de façon connue en soi, l'aldéhyde (I'a), respectivement (Ic), ainsi obtenu pour former l'acétal correspondant.

La réaction d'hydrolyse des esters (IIa) et (IIc) s'effectue dans des conditions classiques, à l'aide d'un acide d'usage courant dans ce type de réactions [voir, par exemple, J. March, Advanced Organic Chemistry, 3rd ed., section 0-11, John Wiley & Sons, USA (1985)]. Les conditions détaillées de ces réactions sont décrites dans les exemples de préparation présentés plus loin. De préférence, on utilise les composés de formule (IIa) ou (IIc) dans laquelle $R^4$ représente un radical méthyle.

Ces derniers sont des composés nouveaux qui font également l'objet de l'invention. Ils peuvent être préparés à partir de dérivés benzéniques d'origine commerciale, en ce qui concerne les composés (IIa), ou, dans le cas des composés (IIc), à partir de l'indane approprié [voir M.T. Bogert et al., J. Amer. Chem. Soc. 56, 185 (1934) ; S.T. Bright et al., J. Org. Chem. 55, 1338 (1990)] par condensation avec un diacétate insaturé [voir, par exemple, I. Scriabine, Bull. Soc. Chim. France 1961, 1194; N.E. Kologrivova et al., C.A. 78, 88513p (1973)].

SCHEMA I

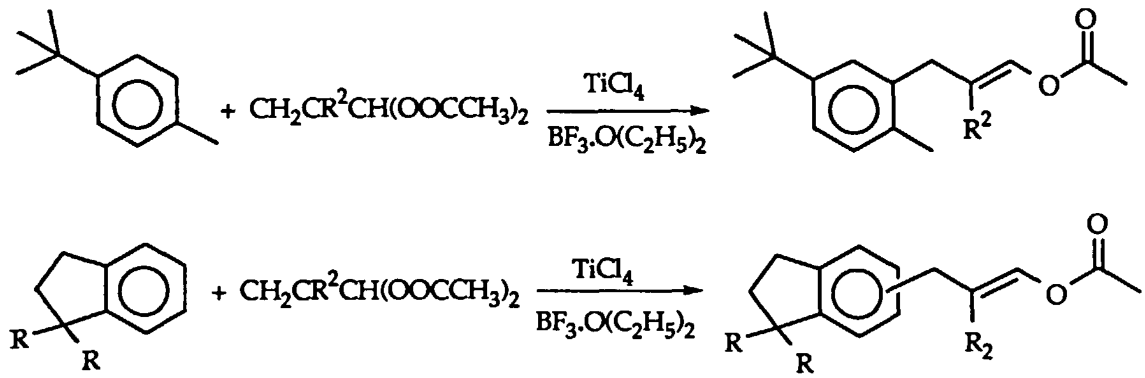


R, $R^2$ = H, $CH_3$

Les conditions de ces réactions de condensation sont décrites plus en détail dans les exemples présentés plus loin.

L'invention concerne aussi un autre procédé pour la préparation d'un composé de formule

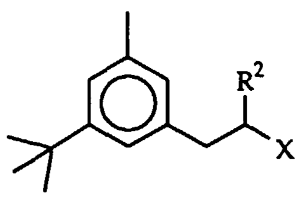

(I"a)

dans laquelle X et $R^2$ sont définis comme à la formule (Ia), caractérisé en ce qu'on soumet à une hydrogénation catalytique, dans un solvant organique inerte, un aldéhyde de formule

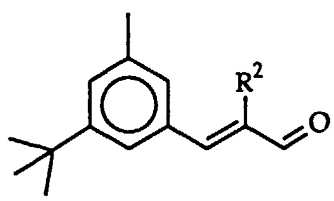

(III)

dans laquelle $R^2$ a le sens indiqué ci-dessus et, le cas échéant, on acétalyse, de façon connue en soi, l'aldéhyde (I"a) ainsi obtenu pour former l'acétal correspondant.

La réaction d'hydrogénation a lieu en présence d'un catalyseur tel que le Pd-C, dans des conditions classiques, décrites plus loin de façon détaillée.

Les produits de départ de formule (III) sont des composés nouveaux, préparés à partir de 5-tert-butyl-1,3-diméthylbenzène, selon le schéma que voici :

SCHEMA II

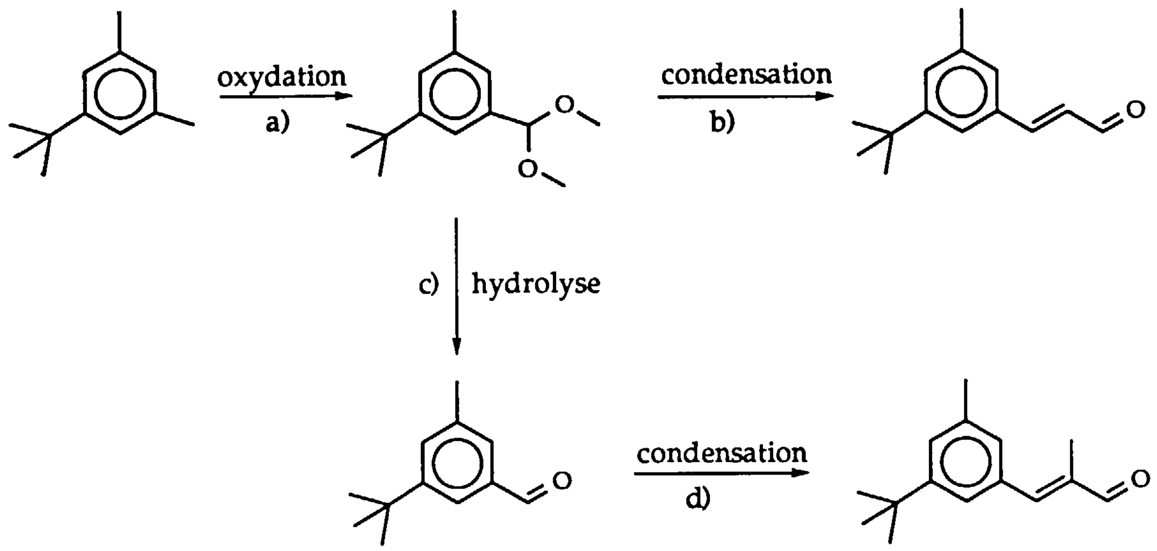


a) -e$^{\ominus}$/C/Inox, p-toluènesulfonate de sodium, $CH_3OH$
b)

i) $CH_3CH_2OCHCH_2$, $ZnCl_2$, $H_3PO_4$, 0-5°C
ii) HCOOH, HCOONa, $H_2O$, 90-110°C

c) HCl 10% aq., THF, t. a.
d) $CH_3CH_2CHO$, $K_2CO_3$, $CH_3OH$, 65°C

Nous avons constaté que la réaction de condensation de l'acétal intermédiaire [étape b), voir, par exemple, H. von der Bruggen et al., J. Org. Chem. 1983, 2920 et réfs y citées] s'effectuait au mieux en présence de chlorure de zinc et d'acide phosphorique.

Les réactions représentées dans ce schéma sont décrites plus en détail dans les exemples présentés plus loin.

Lorsque désiré, les aldéhydes de formule (I'a) et (I"a), obtenus comme il est décrit plus haut, peuvent être transformés en les acétals correspondants à l'aide de méthodes bien connues de l'homme de l'art, par exemple, en faisant réagir lesdits aldéhydes avec des alcools ou des diols appropriés, en présence d'un catalyseur acide [voir, par exemple, J. March, Advanced Organic Chemistry, section 6-6, 3rd ed., John Wiley & Sons, USA (1985)]. Les conditions détaillées de ces réactions d'acétalysation sont décrites dans les exemples de préparation présentés plus loin.

Les composés bicycliques de formule (Ib) selon l'invention sont préparés selon un procédé original caractérisé en ce qu'on traite avec un agent oxydant un alcool de formule

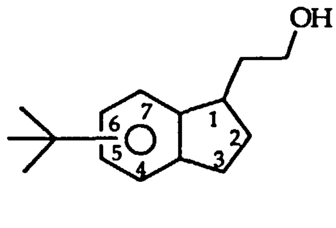 ou 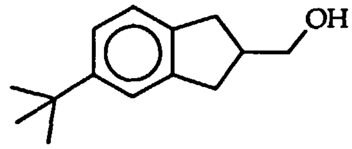

(IVa) (IVb)

et, le cas échéant, on acétalyse de façon connue en soi, l'aldéhyde (Ib) ainsi obtenu pour former l'acétal correspondant.

L'aldéhyde obtenu par oxydation de l'alcool (IVb) peut aussi être méthylé pour fournir un homologue selon l'invention de formule

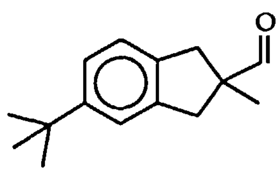

lequel peut ensuite être acétalysé de façon connue en soi.

Les réactions d'oxydation des composés (IV) s'effectuent dans des conditions classiques, par exemple, à l'aide de chlorochromate de pyridinium (PCC) en tant qu'agent oxydant.

Les alcools de formule (IV) sont des composés nouveaux qui peuvent être préparés à l'aide de réactions conventionnelles, comme représenté ci-après pour les composés (IV) ayant le radical tert-butyle en position 5 du cycle aromatique.

SCHEMA III

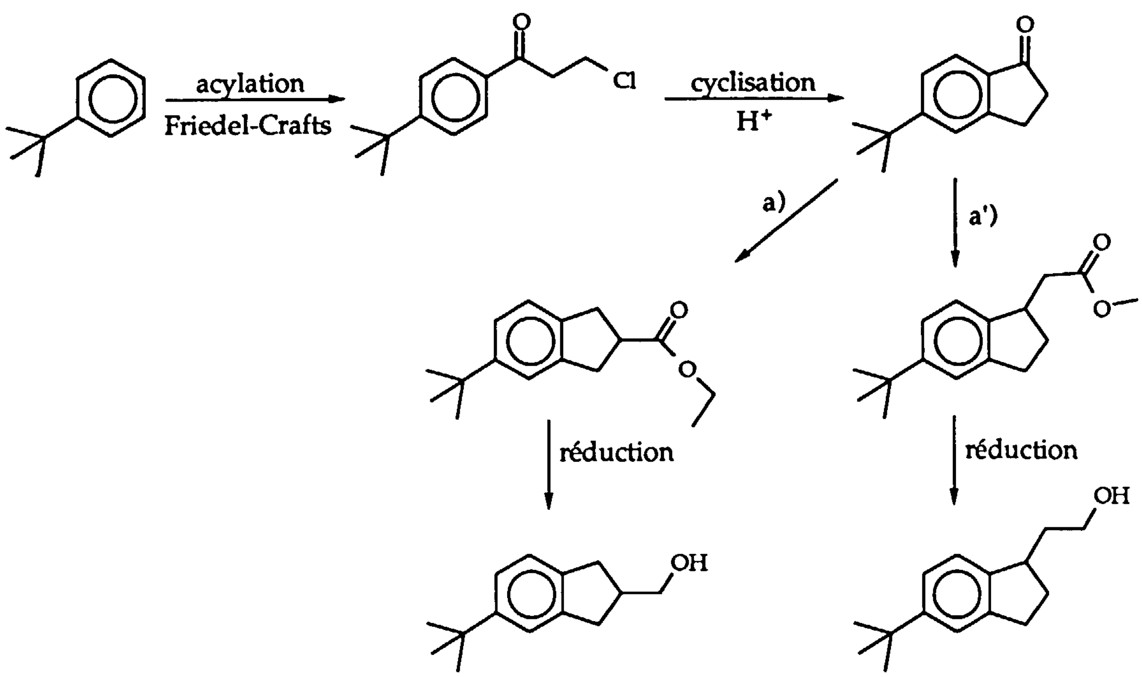


a)

i) NaH, $(CH_3CH_2O)_2CO$, toluène, 60°C
ii) $H_2$, Pd-C 5%, acétate d'éthyle, t. a.

a')

i) $(CH_3O)_2POCH_2COOCH_3$, $NaOCH_3$, éther de pétrole 30-50, t. a.
ii) $H_2$, Pd-C 5%, acétate d'éthyle, t. a.

Ces réactions sont décrites plus en détail dans les exemples de préparation présentés plus loin.
Les aldéhydes (Ib) selon l'invention peuvent ensuite être transformés en les acétals correspondants, comme il est décrit plus haut.
L'invention sera maintenant décrite de façon plus détaillée à l'aide des exemples suivants, dans lesquels les températures sont indiquées en degrés centigrades et les abréviations ont le sens usuel dans l'art.

Exemple 1

Préparation de 3-(5-tert-butyl-2-méthyl-1-phényl)propanal

A une solution d'acétate de 3-(5-tert-butyl-2-méthyl-1-phényl)-1-propényle (mélange Z/E ~ 1:10 ; 0,68 g, 2,7 mmole) dans le tétrahydrofuranne (THF, 8 ml), maintenue sous agitation, on a ajouté $H_2SO_4$ aq. à 25% (2 ml) et le mélange a été chauffé à reflux (65°) pendant 2 h. On a dilué à l'éther et saumure, lavé la phase organique avec $NaHCO_3$ sat. et saumure, séché sur $Na_2SO_4$ et concentré (0,62 g). Après distillation au four à boules (temp. four 160°/0,5x$10^2$ Pa) on a obtenu le 3-(5-tert-butyl-2-méthyl-1-phényl)propanal sous forme d'une huile incolore (0,54g, pureté 96%, rend. 96%).

IR(pur) : 2950, 2890, 2850, 2700, 1720, 1495, 1450, 1355, 1265, 1130, 820 $cm^{-1}$
RMN($^1H$, 360MHz:, $CDCl_3$) : 9,85(t, J=1, 1H); 7,16(dd, $J_1$=8, $J_2$=2, 1H); 7,14 (s large, 1H) ; 7,09(d, J=8, 1H) ; 2,94 (t, J=8, 2H) ; 2,74(t, J=8, 2H) ; 2,28(s, 3H) ; 1,30(s, 9H) δ ppm
RMN($^{13}C$, 90,5MHz, $CDCl_3$) : 201,6(d) ; 149,8(s) ; 138,0(s) ; 132,8(s) ; 130,2(d) ; 125,7(d) ; 123,4(d) ; 44,3(t) ; 34,3 (s) ; 31,4(3q) ; 25,9(t) ; 18,7(q) δ ppm
SM : 204($M^+$,16), 189(94), 171(9), 145(100), 128(16), 115(27), 105(18), 91(25), 77(13), 57(11), 41(13)
Odeur : décrite plus haut.

L'acétate de 3-(5-tert-butyl-2-méthyl-1-phényl)-1-propényle de départ a été préparé ainsi.
A une solution agitée de 4-tert-butyl-toluène (Fluka, pureté 95%, 18,1 ml, 10 mmole) dans $CH_2Cl_2$ (10 ml), à température ambiante, on a ajouté du $TiCl_4$ (Fluka puriss., 1,21 ml, 11 mmole) et du $BF_3.O(C_2H_5)_2$ (30 µl, 1 mmole). La solution orange a été refroidie à -78° et une solution d'acétal de diacétyl acroléine (Fluka purum 98%, 1,66 ml, 11 mmole) dans $CH_2Cl_2$ (5 ml) a été ajoutée goutte à goutte. On a laissé le mélange réactionnel revenir à 0° et agité pendant 15 min à cette température. On a versé sur un mélange de glace, HCl aq. 10% et éther, et lavé la phase organique avec saumure (2 fois), $NaHCO_3$ sat. et saumure. On a séché sur $Na_2SO_4$, concentré (2,52 g) et distillé au four à boules (90°/2 Pa) pour obtenir l'acétate désiré sous forme d'huile jaune (1,64 g, pureté 95%, Z/E ~1:10, rend. 63%).

IR(pur) : 3070, 3015, 2960, 2900, 2860, 1750, 1665, 1365, 1270, 1220, 1185, 1100, 945, 900, 820 $cm^{-1}$
RMN($^1H$, 360MHz, $CDCl_3$) : isomère E
7,20-7,05(m, 4H) ; 5,57(dt, $J_1$=12, $J_2$=7, 1H) ; 3,31(d, J=7, 2H) ; 2,27(s, 3H) ; 2,11(s, 3H) ; 1,30(s, 9H) δ ppm
RMN($^{13}C$, 90,5MHz, $CDCl_3$) : isomère Z
168,1(s) ; 149,1(s) ; 137,2(s) ; 136,4(d) ; 133,0(s) ; 130,0(d) ; 125,8(d) ; 123,4(d) ; 113,4(d) ; 34,4(s) ; 31,6(t) ; 31,4 (3q) ; 20,6(q) ; 18,7(q) δ ppm
SM : 246($M^+$,14), 231(7), 204(8), 189(68), 171(12), 147(27), 129(10), 115(11), 105(8), 91(11), 77(5), 57(28), 43 (100)
Odeur : verte, légumineuse, florale.

Exemple 2

Préparation de 2-(5-tert-butyl-2-méthylbenzyl)propanal

A une solution d'acétate de 3-(5-tert-butyl-2-méthyl-1-phényl)-2-méthyl-1-propényle (26,0 g, 94 mmole, pureté 94%) dans le méthanol (60 ml), maintenue sous agitation, on a ajouté $K_2CO_3$ (1,38 g,10 mmole). La reaction est devenue lentement exothermique et la température du mélange a été maintenue au-dessous de 35° avec un bain d'eau. Après 1 h à 25°, l'analyse CG indiquait la disparition de l'acétate de départ. On a dilué à l'éther, lavé la phase organique avec $NaHCO_3$ sat. et saumure, séché sur $Na_2SO_4$ et concentré pour obtenir une huile jaune (20,8 g). Distillation sur colonne Vigreux de 15 cm, à pression réduite, a fourni le 2-(5-tert-butyl-2-méthylbenzyl)propanal désiré (18,8 g, pureté 89%, rend. 92%).

IR(pur) : 2970, 2910, 2890, 2810, 2710, 1725, 1500, 1460, 1365, 1275, 1145, 825 $cm^{-1}$
RMN($^1H$, 360MHz, $CDCl_3$) : 9,73(d, J=2, 1H) ; 7,16(dd, $J_1$=8, $J_2$=2, 1H) ; 7,12(d, J=2, 1H) ; 7,08(d, J=8, 1H) ; 3,10 (dd, $J_1$=14, $J_2$=6, 1H) ; 2,65(m, 1H) ; 2,56(dd, $J_1$=14, $J_2$=8, 1H) ; 2,28(s, 3H) ; 1,30(s, 9H) ; 1,12(d, J=7, 3H) δ ppm
RMN($^{13}C$, 90,5MHz, $CDCl_3$) : 204.5(d) ; 148,9(s) ; 136,6(s) ; 133,0(s) ; 130,2(d) ; 126,8(d) ; 123,4(d) ; 47,0(d) ; 34,3(s,t) ; 31,4(3q) ; 18,9(q) ; 13,5(q) δ ppm
SM : 218($M^+$,15), 203(72), 185(13), 161(41), 145(100), 131(22), 115(22), 105(26), 91(25), 77(13), 57(28), 41(27)
Odeur : décrite plus haut.

L'acétate de 3-(5-tert-butyl-2-méthyl-1-phényl)-2-méthyl-1-propényle de départ a été préparé ainsi.

A du 4-tert-butyl-toluène (88,8 g, 0,6 mole) à 0°, on a ajouté du $TiCl_4$ (Fluka puriss., 38,0 g, 21,9 ml, 200 mmole) et du $BF_3.O(C_2H_5)_2$ (0,5 ml, 0,56 g, 4 mmole). Le mélange agité a été refroidi à -15° et une solution d'acétal de diacétyl méthacroléine (Fluka purum, 34,4 g, 200 mmole) dans 4-tert-butyl-toluène (30 ml) a été ajoutée goutte à goutte pendant 0,5 h, en maintenant la température entre -10° and -20°. On a agité le mélange réactionnel pendant 30 min entre -10° et +10° et dilué à l'éther. On a lavé avec HCl aq. 10% et saumure (2 fois), $NaHCO_3$ sat. et saumure. On a séché sur $Na_2SO_4$ et concentré au rotavapeur (142 g). Distillation sous vide sur colonne Vigreux de 15 cm a fourni une première fraction contenant 98% de 4-tert-butyl-toluène (98,6 g). La deuxième fraction (temp. bain 180-210°/ $0,6x10^2$ Pa) contenait l'acétate désiré (29,6 g, pureté 86%, Z/E ~ 2:84, rend. 49%).

IR(pur) : 2955, 2900, 2860, 1745, 1675, 1360, 1225, 1100, 1090 $cm^{-1}$
RMN($^1H$, 360MHz, $CDCl_3$) : 7,16(dd, $J_1$=8, $J_2$=2, 1H) ; 7,13(d, J=2, 1H) ; 6,88(s, 1H) ; 3,27(s, 2H) ; 2,26(s, 3H) ; 2,13(s, 3H) ; 1,64(s, 3H) ; 1,30(s, 9H) δ ppm
RMN($^{13}C$, 90MHz, $CDCl_3$) : 168,3(s) ; 148,71(s) ; 136,1(s) ; 133,6(s) ; 130,0(d) ; 126,6(d) ; 123,3(d) ; 120,7(s) ; 38,0(t) ; 34,3(s) ; 31,4(3q) ; 20,8(q) ; 18,9(q) ; 13,9(q) δ ppm
SM : 260($M^+$,15), 218(25), 203(72), 185(13), 161(41), 145(15), 133(30), 115(14), 105(13), 77(6), 57(32), 43(100), 29(11)

Exemple 3

Préparation de 4-tert-butyl-2-(3,3-diméthoxypropyl)-1-méthylbenzène

A une solution de 3-(5-tert-butyl-2-méthyl-1-phényl)propanal (1,06 g, 5 mmole) dans le méthanol (10 ml), à température ambiante, on a ajouté HCl conc. (3 gouttes). Après 3 h, on a versé la solution dans un mélange d'éther et $NaHCO_3$ sat. pour extraction. On a lavé la phase organique avec $NaHCO_3$, séché sur $K_2CO_3$ et concentré (1,25 g). Après distillation au four à boules (120°/6 Pa), on a obtenu le produit désiré, dont la pureté était de 95% (1,2 g, rend. 91%) et lequel présentait les caractères analytiques suivants :

IR(pur) : 2980, 2920, 2890, 2840, 1510, 1465, 1390, 1370, 1280, 1200, 1140, 1090, 1065, 920, 830 $cm^{-1}$
RMN($^1H$, 360MHz, $CDCl_3$) : 7,17(d, J=2, 1H) ; 7,14(dd, $J_1$=8, $J_2$=2, 1H) ; 7,08(d, J=8, 1H) ; 4,42(t, J=6, 1H) ; 3,35 (s, 6H) ; 2,66(m, 2H) ; 2,28(s, 3H) ; 1,89(m, 2H) ; 1,30(s, 9H) δ ppm
RMN($^{13}C$, 90,5MHz, $CDCl_3$) : 148,9(s) ; 139,3(s) ; 132,9(s) ; 130,0(d) ; 125,9(d) ; 122,9(d) ; 104,2(d) ; 52,8(2q) ; 34,3(s) ; 33,1(t) ; 31,5(q) ; 28,6(t) ; 18,7(q) δ ppm
SM : 250($M^+$,< 1), 235(1), 218(14), 203(20), 186(22), 171(56), 161(43), 145(24), 131(43), 106(18), 91(15), 75 (100), 57(22), 41(15)
Odeur : florale, agréable.

Exemple 4

Préparation de 5-tert-butyl-2-indanecarbaldéhyde

A une suspension de chlorochromate de pyridinium (PCC, Fluka, 3,24 g, 15 mmole) dans le dichlorométhane (20 ml), on a ajouté, à température ambiante, une solution de 5-tert-butyl-2-indaneméthanol (2,04 g, 10 mmole) dans

du dichlorométhane (10 ml). Le mélange a été agité pendant 5 h à température ambiante. On a dilué à l'éther (50 ml), filtré sur CELITE®, ensuite sur colonne de FLORISIL® (Fluka), et concentré. La distillation au four à boules a fourni le 5-tert-butyl-2-indanecarbaldéhyde (1,47 g, pureté > 99%, rend. 72%) sous forme d'une huile incolore.

RMN($^1$H, 360MHz, $CDCl_3$) : 9,77(d, J=2, 1H) ; 7,27(s, 1H) ; 7,22(d, J=8, 1H) ; 7,16(d, J=8, 1H) ; 3,35-3,10(m, 5H) ; 1,31(s, 9H) δ ppm
RMN($^{13}$C, 90,5MHz, $CDCl_3$) : 203,0(d) ; 150,1(s) ; 141,0(s) ; 138,1(s) ; 124,1(d) ; 124,0(d) ; 121,5(d) ; 50,9(d) ; 34,6(s) ; 33,1(t) ; 32,5(t) ; 31,5(q) δ ppm
SM : 202($M^+$,36), 187(100), 169(10), 157(10), 141(11), 129(18), 115(19), 91(6), 77(3), 57(6), 41(6)
Odeur : décrite plus haut.

L'alcool de départ a été préparé selon le Schéma III, comme suit.

a) 5-tert-butyl-1-indanone

A une solution de tert-butylbenzène (88,9 g, 0,7 mole) et de chlorure de 3-chloropropionyle (Fluka, 93,8 g, 0,7 mole) dans $CH_2Cl_2$ (105 ml), à 0°, on a ajouté par portions du $AlCl_3$ (95,7g, 0,7 mole) pendant 2 h. Après 3 h à 0°, le mélange a été versé sur de la glace et dilué avec $CH_2Cl_2$ (200 ml). La phase organique a été lavée à l'eau (2 fois) et concentrée. Le résidu a été dissous à l'éther, lavé avec $NaHCO_3$ aq. sat. et saumure, séché sur $Na_2SO_4$ et concentré (152 g). On a cristallisé dans l'éther de pétrole 30-50°, à -30°, pour obtenir des cristaux incolores (117 g, pureté > 99%, rend. 52%) de 1-(4-tert-butyl-1-phényl)-3-chloro-1-propanone.
P.f. 35-37°

RMN($^1$H, 360MHz, $CDCl_3$) : 7,90(d, J=8, 2H) ; 7,49(d, J=8, 2H); 3,92(t, J=7, 2H) ; 3,43(t, J=7, 2H) ; 1,34(s, 9H) δ ppm
RMN($^{13}$C, 90,5MHz, $CDCl_3$) : 196,2(s) ; 157,3(s) ; 133,7(s) ; 128,0(2d) ; 125,7(2d) ; 41,1(t) ; 38,8(t) ; 35,1(s) ; 31,0(3q) δ ppm
SM : 224($M^+$,4), 209(13), 173(9), 161(100), 146(7), 118(9), 91(11), 77(4), 63(5), 41(2)

Cette propanone (116 g, 0,51 mole) a été dissoute dans $H_2SO_4$ conc. (920 ml) et chauffée à 100° sous agitation (dégagement de HCl).
Après 1,5 h, on a refroidi et versé sur un mélange de glace (3,0 kg), NaCl (230 g) et éther (400 ml), lavé la phase organique avec $H_2O$, $NaHCO_3$ aq. sat. et saumure, séché sur $Na_2SO_4$ et évaporé (97g). On a cristallisé dans l'éther de pétrole 30-50°, à -30°, pour obtenir des cristaux incolores (74 g, pureté > 99%) de 5-tert-butyl-1-indanone.
P.f. 39-40°

IR($CHCl_3$) : 2960, 2880, 1708, 1603, 1325, 1085, 710 $cm^{-1}$
RMN($^1$H, 360MHz, $CDCl_3$) : 7,69(d, J=8, 1H) ; 7,48(s, 1H) ; 7,43(d, J=8, 1H) ; 3,12(t, J=6, 2H) ; 2,67(m, 2H) ; 1,36(s, 9H) δ ppm
RMN($^{13}$C, 90,5MHz, $CDCl_3$) : 206,5(s) ; 158,8(s) ; 155,5(s) ; 134,8(s) ; 125,0(d) ; 123,3(d) ; 123,2(d) ; 36,5(t) ; 35,4(s) ; 31,2(q) ; 25,9(t) δ ppm
SM : 188($M^+$,31), 173(100), 145(23), 131(29), 115(12), 103(3), 91(8), 77(5), 51(3), 41(3)
Odeur : vaguement crésolique.

b) 5-tert-butyl-2-indanecarboxylate d'éthyle

Une dispersion de NaH (80% dans huile, 4,32 g, 144 mmole NaH) a été lavée au pentane et on lui a ajouté du toluène (140 ml) et carbonate de diéthyle (34 g, 288 mmole). On a chauffé le mélange à 60° et on a ajouté, pendant 2 h, une solution dans le toluène (20 ml) de l'indanone préparée en a) (7,52 g, 40 ml). Le mélange réactionnel a été maintenu sous agitation à 60° pendant 6 h.

On a versé sur un excès de $H_2O/CH_3COOH$ 1:1 et extrait à l'éther de pétrole 30-50°, lavé avec $NaHCO_3$ aq. sat. et saumure, séché sur $Na_2SO_4$ et concentré (10,8 g). Après distillation au four à boules (150°/10 Pa), on a obtenu 7,12g de 5-tert-butyl-1-oxo-2-indanecarboxylate d'éthyle.

IR(pur) : 2960, 2860, 1730-1700, 1598, 1360, 1320, 1250, 1205, 1150, 1080, 1010 $cm^{-1}$
RMN($^1$H, 360MHz, $CDCl_3$) : 77,0(d, J=8, 1H) ; 7,50(s, 1H) ; 7,45(d, J=8, 1H) ; 4,25(q, J=7, 2H) ; 3,71(dd, $J_1$=8, $J_2$=4, 1H) ; 3,54(dd, $J_1$=17, $J_2$=4, 1H) ; 3,35(dd, $J_1$=17, $J_2$=8, 1H) ; 1,36(s, 9H) ; 1,32(t, J=7,3H) δ ppm
RMN($^{13}$C, 90,5MHz, $CDCl_3$) : 199,2(s) ; 169,4(s) ; 159,8(s) ; 154,0(s) ; 132,9(s) ; 125,6(d) ; 124,3(d) ; 123,0 (d) ; 61,6(t) ; 53,6(d) ; 35,5(s) ; 31,1(3q) ; 30,4(t) ; 14,2(q) δ ppm
SM : 260($M^+$,61), 245(25), 215(24), 199(60), 186(77), 171(100), 157(16), 143(11), 131(34), 115(31), 91(18),

57(76), 41(31)

Une solution de ce composé (4,68 g, 16,2 mmole) dans l'acétate d'éthyle (50 ml) a été agitée à température ambiante, en présence de 5% Pd/C (0,48 g) sous $H_2$ (1 atm) pendant 12 h. On a filtré le catalyseur sur CELITE® et concentré la solution (4,25 g). On a chromatographié sur $SiO_2$ (106 g) avec pentane/éther 9:1 en tant qu'éluant et distillé au four à boules (160°/5 Pa) pour obtenir le 5-tert-butyl-2-indanecarboxylate d'éthyle désiré (3,47g, pureté > 99%, rend. 86%).

RMN($^1$H, 360MHz, $CDCl_3$) : 7,24(s, 1H) ; 7,20(d, J=8, 1H) ; 7,13(d, J=8, 1H) ; 4,18(q, J=7, 2H) ; 3,40-3,10(m, 5H) ; 1,31(s, 9H) ; 1,28(t, J=7, 3H) δ ppm
RMN($^{13}$C, 90,5MHz, $CDCl_3$) : 175,4(s) ; 149,8(s) ; 141,5(s) ; 138,7(s) ; 123,8(d) ; 123,7(d) ; 121,2(d) ; 60,6(t) ; 43,8(t) ; 36,3(t) ; 35,8(t) ; 34,5(s) ; 31,6(3q) ; 14,3(q) δ ppm
SM : 246($M^+$,25), 231(100), 201(3), 172(25), 157(60), 129(20), 115(20), 91(4), 79(4), 57(20), 41(7)
Odeur : florale, muguet, hydroxycitronellal.

c) 5-tert-butyl-2-indaneméthanol

A une suspension de $LiAlH_4$ (0,27 g, 7,2 mmole) dans l'éther (10 ml), à température ambiante, on a ajouté une solution du carboxylate obtenu selon b) (2,2 g, 8,9 mole) dans l'éther (10 ml). On a laissé réagir sous agitation pendant 2 h, à température ambiante. On a dilué à l'éther, versé sur $H_2O$ et lavé la phase organique avec HCl aq. 10%, $H_2O$, $NaHCO_2$ sat. et saumure. On a séché sur $Na_2SO_4$, concentré et distillé au four à boules (200°/40 Pa) pour obtenir l'alcool susmentionné sous forme d'huile incolore (1,86 g, pureté > 99%, rend. 100%).

RMN($^1$H, 360MHz, $CDCl_3$) : 7,24(s, 1H) ; 7,18(d, J=8, 1H) ; 7,13(d, J=8, 1H) ; 3,66(d, J=6, 2H) ; 3,04(m, 2H) ; 2,71(m, 3H) ; 1,62(s large, 0H) 1,31(s, 9H) δ ppm
RMN($^{13}$C, 90,5MHz, $CDCl_3$) : 149,5(s) ; 142,5(s) ; 139,7(s) ; 124,1(d) ; 123,4(d) ; 121,5(d) ; 66,7(t) ; 41,7(d) ; 39,9(t) ; 35,3(t) ; 34,5(s) ; 31,6(3q) δ ppm
SM : 204($M^+$,21), 189(100), 171(15), 143(14), 129(16), 115(13), 91(6), 77(3), 57(7), 41(5)

Exemple 5

Préparation de 5-tert-butyl-2-méthyl-2-indanecarbaldéhyde

A une solution de tert-butoxyde de potassium (Fluka, 0,45 g, 4 mmole) et de 5-tert-butyl-2-indanecarbaldéhyde (ex. 4 ; 0,71 g, 3,5 mmole), on a ajouté, à température ambiante et sous $N_2$, de l'iodure de méthyle (0,56 g, 4 mmole) et laissé réagir à température ambiante, sous agitation. On a repris à l'éther et lavé avec $NH_4Cl$ et saumure, séché sur $Na_2SO_4$ et concentré (0,9 g). On a chromatographié sur $SiO_2$ (30 g), avec toluène comme éluant, pour obtenir 50 mg (pureté > 99%, rend. 7%) de l'aldéhyde désiré.

RMN($^1$H, 360MHz, $CDCl_3$) : 9,65(s, 1H) ; 7,23(s, 1H) ; 7,22(d, J=8, 1H) ; 7,13(d, J=8, 1H) ; 3,36(d, J=16, 1H) ; 3,33(d, J=16, 1H) ; 2,76(d, J=16, 1H) ; 2,74(d, J=16, 1H); 1,31(s, 9H) ; 1,30(s, 3H) δ ppm
RMN($^{13}$C, 90,5MHz, $CDCl_3$) : 204,1(d) ; 150,1(s) ; 140,8(s) ; 137,9(s) ; 124,2(d) ; 124,0(d) ; 121,6(d) ; 54,3(s) ; 41,1(t) ; 40,6(t) ; 34,6(s) ; 31,5(3q) ; 21,1(q) δ ppm
SM : 216($M^+$,37), 201(100), 183(4), 171(5), 157(16), 141(9), 129(18), 115(5), 91(7), 71(6), 57(24), 41(8)
Odeur : décrite plus haut.

Exemple 6

Préparation de 2-(5-tert-butyl-2-indanyl)-1,3-dioxolane

Un mélange de 5-tert-butyl-2-indanecarbaldéhyde (ex. 4 ; 9,8 mmole), d'éthylèneglycol (6,1 g, 98 mmole) et d'acide p-toluènesulfonique (95 mg, 0,5 mmole) dans le cyclohexane (25 ml), a été chauffé à reflux (80°) pendant 3 h avec une trappe de type Dean-Stark. Le mélange refroidi a été versé sur éther et $NaHCO_3$ aq. sat., et la phase organique lavée avec $NaHCO_3$ aq. sat., séchée sur $K_2CO_3$ et concentrée. Après distillation au four à boules, on a obtenu le dioxolane désiré.

IR : 2980, 2890, 1500, 1400, 1370, 1275, 1210, 1155, 1130, 1080, 1050, 980, 950, 925, 830 $cm^{-1}$

RMN($^1H$, 360MHz, $CDCl_3$) : 7,24(s, 1H) ; 7,18(d, J=8, 1H) ; 7,13(d, J=8, 1H) ; 4,87(d, J=6, 1H) ; 4,0(m, 2H) ; 3,88 (m, 2H) ; 3,10-2,85(m, 4H) ; 2,71(m, 1H) ; 1,31(s, 9H) δ ppm
RMN($^{13}C$, 90,5MHz, $CDCl_3$) : 149,5(s) ; 142,4(s) ; 139,6(s) ; 124,0(d) ; 123,4(d) ; 121,4(d) ; 106,9(d) ; 65,1(t) ; 43,0 (d) ; 34,5(tz) ; 33,9(t); 31,6(3q) δ ppm
SM : 246($M^+$,16), 231(6), 184(30), 169(15), 157(9), 141(8), 128(13), 115(13), 73(100), 57(16), 45(27), 41(8), 29(9)
Odeur : florale.

Exemple 7

Préparation de 5-tert-butyl-1-indaneacétaldéhyde

Préparé de façon analogue à celle décrite dans l'Exemple 4, par oxydation de 2-(5-tert-butyl-1-indanyl)-1-éthanol (3,85 g, 17,6 mmole) dans $CH_2Cl_2$ (40 ml), à l'aide de PCC (5,73 g, 25 mmole) dans $CH_2Cl_2$ (60 ml). Après le traitement décrit et distillation au four à boules (150°/40 Pa), on a obtenu l'acétaldéhyde en titre (2,34 g, rend. 62%).

IR(pur) : 2960, 2900, 2860, 2710, 1725, 1490, 1360, 1260, 830 $cm^{-1}$
RMN($^1H$, 360MHz, $CDCl_3$) : 9,88(t, J=2, 1H) ; 7,28(s, 1H) ; 7,22(d, J=8, 1H) ; 7,08(d, J=8, 1H) ; 3,62(m, 1H) ; 2,95-2,85(m, 2H) ; 2,62(ddd, $J_1$=17, $J_2$=9, $J_3$=2, 1H) ; 2,43(m, 1H) ; 1,70(m, 1H) ; 1,32(s, 9H) δ ppm
RMN($^{13}C$, 90,5MHz, $CDCl_3$) : 202,2(d) ; 150,1(s) ; 143,6(s) ; 142,5(s) ; 123,5(d) ; 122,9(d) ; 121,5(d) ; 49,5(d) ; 38,6(d) ; 34,6(s) ; 32,7(t) ; 31,6(3q) ; 31,5(t) δ ppm
SM : 216($M^+$,44), 201(111), 173(79), 157(98), 143(37), 129(72), 115(73), 102(5), 91(23), 77(10), 57(33), 41(8), 29 (8)
Odeur : métallique, florale.

Le 2-(5-tert-butyl-1-indanyl)-1-éthanol de départ a été préparé à partir de 5-tert-butyl-1-indanone [ex. 4 a)], comme suit.

a) 5-tert-butyl-1-indaneacétate de méthyle

A une suspension d'hydrure de sodium (3,6 g d'une dispersion à 50% dans l'huile, 75 mmole) préalablement lavé avec de l'éther de pétrole 30-50, dans 100 ml de tétrahydrofurane (THF), on a ajouté goutte à goutte à 10-20° 13,8 g (75 mmole) de triméthylphosphonoacétate dans 100 ml de THF. 15 min après la fin de l'addition, on a ajouté au mélange de réaction, à 11-15°, une solution de l'indanone citée ci-dessus (10,2 g, 55 mmole) dans le THF (150 ml). Le mélange a été maintenu sous agitation pendant 16 h à 25°, puis il a été versé dans 500 ml d'eau et extrait à l'éther. Les extraits organiques combinés ont été lavés avec saumure (2 fois), séchés ($Na_2SO_4$) et concentrés jusqu'à former une huile qui est devenue solide (12,6 g ; mélange d'isomères). On a cristallisé ce produit dans l'éther, à -30°, pour obtenir 5,56 g de (E)-(5-tert-butyl-1-indanylidène)acétate de méthyle.
P.f. 116,5-117,5°

IR($CHCl_3$) : 2960, 1690, 1630, 1605, 1435, 1355, 1315, 1290, 1175, 1090, 830 $cm^{-1}$
RMN($^1H$, 360MHz, $CDCl_3$) : 7,53(d, J=8, 1H) ; 7,37(s, 1H) ; 7,30(d, J=8, 1H) ; 6,27(t, J=2,5, 1H) ; 3,76(s, 3H) ; 3,29(m, 2H) ; 3,06(t, J=6, 2H) ; 1,33(s, 9H) δ ppm
RMN($^{13}C$, 90,5MHz, $CDCl_3$) : 168,1(s) ; 163,4(s) ; 154,8(s) ; 149,7(s) ; 137,4(s) ; 124,4(d) ; 122,2(d) ; 121,3 (d) ; 106,4(d) ; 50,9(q) ; 35,0(s) ; 31,5(t) ; 31,3(3q) ; 30,7(t) δ ppm
SM : 244($M^+$,43), 229(100), 213(15), 197(14), 188(9), 169(15), 155(27), 141(25), 128(34), 115(23), 85(10), 57(13), 41(7)

Une solution de ce composé (7,4 g, pureté 94%, 30 mmole) dans l'acétate d'éthyle (150 ml) a été hydrogénée en présence de 5% Pd/C (0,68 g, 0,3 mmole) pendant 1 h. Après filtration du catalyseur sur CELITE® et concentration de la solution (7,93 g), on a distillé au four à boules (110°/35 Pa) pour obtenir le 5-tert-butyl-1-indaneacétate de méthyle (7,1 g, pureté > 99%, rend. 93%).

IR(pur) : 2970, 1735, 1435, 1360, 1270, 1190, 1170, 830 $cm^{-1}$
RMN($^1H$, 360MHz, $CDCl_3$) : 7,26(s, 1H) ; 7,20(d, J=8, 1H) ; 7,00(d, J=8, 1H) ; 3,72(s, 3H) ; 3,55(m, 1H) ; 3,0-2,7(m, 3H) ; 2,5-2,3(m, 2H) ; 1,73(m, 1H) ; 1,32(s, 9H) δ ppm
RMN($^{13}C$, 90,5MHz, $CDCl_3$) : 173,3(s) ; 149,9(s) ; 143,7(s) ; 142,7(s) ; 123,4(d) ; 122,9(d) ; 121,5(d) ; 51,5 (q) ; 41,0(d) ; 39,7(t) ; 34,5(s) ; 32,7(t) ; 31,6(3q) ; 31,3(t) δ ppm
SM : 246($M^+$,28), 231(58), 189(52), 173(74), 157(65), 143(32), 129(100), 115(54), 91(15), 77(7), 57(31), 41(7)

b) 2-(5-tert-butyl-1-indanyl)-1-éthanol

Préparée à partir de l'ester décrit sous a) (6,3 g, 25 mmole), de façon analogue à celle décrite dans l'ex. 4 c), à l'aide de $LiAlH_4$ (38 mmole). On a ajouté 2 ml d'acétate d'éthyle au mélange de la réaction, ensuite 4,85 ml NaOH aq. 1N et agité pendant 30 min à température ambiante. Après addition de $Na_2SO_4$ et filtration, on a lavé à l'éther et évaporé (5,5 g). La distillation au four à boules (115°/30 Pa) a fourni l'alcool désiré (5,5 g, pureté > 99%, rend. 99%).

IR(pur) : 3320, 2960, 2860, 1490, 1360, 1265, 1060, 1025, 825 $cm^{-1}$
RMN($^1H$, 360MHz, $CDCl_3$) : 7,27(s, 1H) ; 7,21(d, J=8, 1H) ; 7,14(d, J=8, 1H) ; 3,81(m, 2H) ; 3,2(m, 1H) ; 3,0-2,8 (m, 2H) ; 2,32(m, 1H) ; 21,5(m, 1H) ; 1,70(m, 2H) ; 1,32(s, 9H) $\delta$ ppm
RMN($^{13}C$, 90,5MHz, $CDCl_3$) : 149,6(s) ; 144,0(s) ; 143,7(s) ; 123,2(d) ; 123,0(d) ; 121,4(d) ; 61,7(t) ; 41,1(d) ; 38,0(t) ; 34,5(s) ; 32,5(t) ; 31,6(3q) $\delta$ ppm
SM : 218($M^+$,56), 203(99), 200(3), 185(25), 173(100), 161(34), 143(30), 128(22), 115(20), 91(8), 77(4), 57 (25), 41(6), 31(9)

Exemple 8

Préparation de 3-(3-tert-butyl-5-méthylphényl)propanal

On a ajouté à 1,2 g (5,7 mmole) de (E)-3-(3-tert-butyl-5-méthylphényl)-2-propénal dans l'éthanol (20 ml) du Pd/C à 5% (0,12 g) et de l'acétate de sodium (0,12 g, 1,4 mmole). Le mélange a été hydrogéné à température ambiante et à pression atmosphérique pendant 6 h. Après filtration du catalyseur, on a évaporé le solvant (1,2 g). Ce produit brut a été dissous dans $CH_2Cl_2$ (20 ml) et ajouté à température ambiante à une suspension de PCC (0,62 g, 2,8 ml) dans $CH_2Cl_2$ (20 ml). Après 2 h, on a dilué à l'éther (100 ml) filtré sur colonne de FLORISIL® (Fluka) et concentré (1,2 g). On a distillé au four à boules (80°/10 Pa) pour obtenir le 3-(3-tert-butyl-5-méthylphényl) propanal (6,88 g, pureté > 95%, rend. 70%).

IR(pur) : 2960, 2860, 1720, 1595, 1470, 1360, 1220, 860, 710 $cm^{-1}$
RMN($^1H$, 360MHz, $CDCl_3$) : 9,82(t, J=1,5, 1H) ; 7,06(s, 1H) ; 7,02(s, 1H) ; 6,83(s, 1H) ; 2,92(t, J=8, 2H) ; 2,76(dt, $J_1$=1,5, $J_2$=8, 2H) ; 2,32(s, 3H) ; 1,30(s, 9H) $\delta$ ppm
RMN($^{13}C$, 90,5MHz, $CDCl_3$) : 201,8(d) ; 151,5(s) ; 139,9(s) ; 137,5(s) ; 126,2(d) ; 124,2(d) ; 122,4(d) ; 45,5(t) ; 34,5 (s) ; 31,4(3q) ; 28,3(t) ; 21,6(q) $\delta$ ppm
SM : 204($M^+$,33), 189(42), 161(49), 145(100), 133(25), 119(23), 105(20), 91(18), 77(9), 65(4), 57(18), 41(6), 29(5)
Odeur : décrite plus haut.

L'aldéhyde insaturé de départ a été préparé selon le schéma II, comme suit.
Le 5-tert-butyl-1,3-diméthylbenzène (Fluka, 190 g, 1,17 mole) a été oxydé électrochimiquement (anode : graphite ; cathode : inox ; densité de courant : 30 $mA/cm^2$ ; 4 F/mole) à une température d'environ 35° en solution dans le méthanol, en utilisant du p-toluènesulfonate de sodium comme électrolyte. Après distillation (Vigreux), on a obtenu 77,3 g de 1-tert-butyl-3-(diméthoxyméthyl)-5-méthylbenzène, d'une pureté de 87% (rend. 26%).
P. éb. 118-128°/11x$10^2$ Pa

IR(pur) : 2980, 2920, 2840, 1745, 1605, 1455, 1370, 1235, 1200, 1175, 1120, 1090, 1070, 1000, 920, 870, 715 $cm^{-1}$
RMN($^1H$, 360MHz, $CDCl_3$) : 7,25(s, 1H) ; 7,17(s, 1H) ; 7,08(s, 1H) ; 5,35(s, 1H) ; 3,34(s, 6H) ; 2,35(s, 3H) ; 1,32 (s, 9H) $\delta$ ppm
RMN($^{13}C$, 90,5MHz, $CDCl_3$) : 151,2(s) ; 137,7(s) ; 137,4(s) ; 126,3(d) ; 124,5(d) ; 120,7(d) ; 103,9(d) ; 52,9(2q) ; 34,6(s) ; 31,4(3q) ; 21,6(q) $\delta$ ppm
SM : 222($M^+$,2), 207(3), 191(100), 177(5), 161(9), 133(9), 115(6), 105(8), 91(8), 75(10), 57(3), 41(2)

On a ajouté à l'acétal ainsi obtenu (12,7 g, 50 mmole), à -10°, rapidement et sous agitation, une solution de $ZnCl_2$ (0,27 g, 2 mmole) dans l'acétate d'éthyle (2,7 ml). Après 5 min, on a ajouté du $H_3PO_4$ à 85% (0,24 ml, 3,5 mmole). Après 15 min, on a ajouté goutte à goutte de l'éthylvinyléther (Fluka purum, 7,3 ml, 75 mmole) en maintenant la température entre -10 et 0°. Après 2,5 h à 0° et 15 h à température ambiante, l'analyse chromatographique indiquait la formation de 65% des acétals intermédiaires.

Ce mélange brut d'acétals a été ajouté au moyen d'une canule à un mélange d'acide formique (12,5 ml), formiate de sodium (4 g) et eau (6,5 ml), et le tout chauffé avec un bain à 110°, en distillant continuellement les volatiles (p. éb. 80-90°/$10^6$ Pa), pendant 1 h. Le résidu a été dilué à l'eau (40 ml) et éther de pétrole 30-50° (40 ml) pour extraction. On a lavé la phase organique, séché et concentré. Le produit brut (12,1 g, pureté 66%, rend. 79%) a été

distillé sur colonne Vigreux (5 cm) pour fournir le (E)-3-(3-tert-butyl-5-méthylphényl)-2-propénal (6,4 g).
P. éb. 70-82°/10 Pa.

IR(pur) : 2960, 2860, 1670, 1620, 1590, 1475, 1360, 1240, 1125, 970, 700 $cm^{-1}$
RMN($^1H$, 360MHz, $CDCl_3$) : 9,69(d, J=8, 1H) ; 7,46(d, J=15, 1H) ; 7,38(s, 1H) ; 7,29(s, 1H) ; 7,22(s, 1H) ; 6,72(dd, $J_1$=16, $J_2$=8, 1H) ; 2,38(s, 3H) ; 1,33(s, 9H) δ ppm
RMN($^{13}C$, 90,5MHz, $CDCl_3$) : 193,8(d) ; 157,7(d) ; 152,1(s) ; 138,4(s) ; 133,7(s) ; 129,5(d) ; 128,2(d) ; 126,2(d) ; 123,1(d) ; 34,6(s) ; 31,2(3q) ; 21,5(q) δ ppm
SM : 202($M^+$,12), 187(25), 159(13), 145(100), 128(12), 115(16), 105(4), 91(8), 79(4), 65(3), 55(3), 41(3)

Exemple 9

Préparation de 3-(3-tert-butyl-5-méthylphényl)-2-méthylpropanal

Préparé par hydrogénation du 3-(3-tert-butyl-5-méthylphényl)-2-méthyl-2-propénal (2,32 g, 8,5 mmole), de façon analogue à celle décrite dans l'Exemple 8 (réaction 2,5 h). Après le traitement décrit et distillation au four à boules (90-105°/10 Pa), on a obtenu 1,5 g de produit d'une pureté de 86% (rend. 78%). La purification sur colonne de $SiO_2$, avec $CH_2Cl_2$ en tant qu'éluant, a fourni le propanal désiré d'une pureté de 94%.

IR(pur) : 2970, 2870, 1725, 1600, 1475, 1450, 1360, 1225, 860, 715 $cm^{-1}$
RMN($^1H$, 360MHz, $CDCl_3$) : 9,72(d, J=2, 1H) ; 7,05(s, 1H) ; 6,98(s, 1H) ; 6,80(s, 1H) ; 3,04(dd, $J_1$=13, $J_2$=6, 1H) ; 2,64(m, 1H) ; 2,56(dd, $J_1$=13, $J_2$=8, 1H) ; 2,32(s, 3H) ; 1,30(s, 9H) ; 1,08(d, J=7, 3H) δ ppm
RMN($^{13}C$, 90,5MHz, $CDCl_3$) : 204,6(d) ; 151,4(s) ; 138,4(s) ; 137,6(s) ; 126,9(d) ; 124,2(d) ; 123,2(d) ; 48,1(d) ; 36,9(t) ; 34,5(s) ; 31,4(3q) ; 21,6(q) ; 13,3(q) δ ppm
SM : 218($M^+$,32), 203(20), 190(11), 175(100), 161(35), 145(80), 133(57), 115(28), 105(45), 91(28), 77(14), 57(32), 41(22), 29(21)
Odeur : décrite plus haut.

L'aldéhyde insaturé de départ a été préparé à partir de 1-tert-butyl-3-(diméthoxyméthyl)-5-méthylbenzène (voir ex. 8), selon le schéma II, comme suit.
A une solution de l'acétal susmentionné (22,2 g, 90 mmole) dans THF (50 ml), on a ajouté de l'HCl aq. 10% (10 ml) et agité le mélange pendant 1,5 h. Après le traitement usuel et distillation sous vide ($10^3$ Pa), on a obtenu 2 fractions, dont la plus pure (11,2 g) contenait du 3-tert-butyl-5-méthylbenzènaldéhyde d'une pureté de 95%.
P. éb. 112-114°/$10^3$ Pa

IR(pur) : 2980, 2880, 1700, 1600, 1480, 1370, 1305, 1235, 1170, 1160, 875, 710 $cm^{-1}$
RMN($^1H$, 360MHz, $CDCl_3$) : 9,98(s, 1H) ; 7,71(s, 1H) ; 7,50(s, 1H) ; 7,47(s, 1H) ; 2,43(s, 3H) ; 1,35(s, 9H) δ ppm
RMN($^{13}C$, 90,5MHz, $CDCl_3$) : 192,9(d) ; 152,2(s) ; 138,5(s) ; 136,5(s) ; 132,6(d), 127,8(d) ; 123,9(d) ; 34,7(s) ; 31,2 (3q) ; 21,4(q) δ ppm
SM : 176($M^+$,26), 161(100), 145(2), 133(31), 115(16), 105(32), 91(18), 77(9), 65(7), 51(5), 41(11), 29(7)

A une solution de ce benzaldéhyde (3,55 g, 18 mmole) dans le méthanol (20 ml), on a ajouté du $K_2CO_3$ (2,76 g, 20 mmole) et chauffé le mélange à reflux (65°). On a ajouté goutte à goutte, pendant 1 h, une solution de propanal (1,71 g, 24 mmole) dans le méthanol (5 ml). Après avoir chauffé à reflux pendant encore 1 h, on a refroidi et dilué à l'éther, lavé avec $NaHCO_3$ aq. sat. et saumure, séché sur $Na_2SO_4$ et concentré (3,75 g). Après distillation au four à boules (125°/10 Pa), on a obtenu le 3-(3-tert-butyl-5-méthylphényl)-2-méthyl-2-propénal sous forme d'une huile incolore (1,83 g, pureté 79%, rend. 57%).

IR(pur) : 2970, 2930, 2870, 1675, 1622, 1595, 1480, 1445, 1400, 1365, 1280, 1230, 1195, 1030, 920, 835, 705 $cm^{-1}$
RMN($^1H$, 360MHz, $CDCl_3$) : 9,58(s, 1H) ; 7,35(s, 1H) ; 7,25(s, 1H) ; 7,18(s, 1H) ; 2,40(s, 3H) ; 2,09(s, 3H) ; 1,35 (s, 9H) δ ppm
RMN($^{13}C$, 90,5MHz, $CDCl_3$) : 195,7(d) ; 151,6(s) ; 150,8(d) ; 138,0(2s) ; 134,9(s) ; 127,8(d) ; 127,7(d) ; 124,5(d) ; 34,6(s) ; 31,3(3q) ; 21,7(q) ; 11,0(q) δ ppm
SM : 216($M^+$,8), 201(20), 173(18), 159(100), 145(8), 131(19), 128(11), 115(12), 105(7), 91(7), 77(4), 57(3), 41(3), 29(2)

Exemple 10

Préparation de 3-(3,3-diméthyl-5-indanyl)propanal et de 3-(1,1-diméthyl-5-indanyl)propanal

Préparé par hydrolyse de l'acétate de (E)-3-(3,3-diméthyl-5-indanyl)-1-propényle [1,93 g, 7,4 mmole ; mélange contenant 58% de ce composé et 25% de son isomère (1,1-diméthyl-5-indanyl)] à l'aide de $H_2SO_4$ (24 ml) de façon analogue à celle décrite dans l'Exemple 1. Après distillation au four à boules (100°/11 Pa), on a obtenu une huile incolore (1,43 g, rend. 90%, pureté 95%) contenant 58% de 3-(3,3-diméthyl-5-indanyl)propanal et 28% de 3-(1,1-diméthyl-5-indanyl)propanal.
IR(pur) : 2960, 2860, 2720, 1728, 1490, 1450, 1360, 825 $cm^{-1}$
Le 3-(3,3-diméthyl-5-indanyl)propanal présentait les caractères analytiques suivants :

RMN($^1$H, 360MHz, $CDCl_3$) : 9,82(t, J=15, 1H) ; 7,10(d, J=8, 1H) ; 6,96(d, J=8, 1H) ; 6,95(s, 1H) ; 2,94(t, J=7, 2H) ; 2,84(t, J=7, 2H) ; 2,75(t, J=7, 2H) ; 1,91(t, J=7, 2H) ; 1,24(s, 6H) δ ppm
RMN($^{13}$C, 90,5MHz, $CDCl_3$) : 201,8(d) ; 153,1(s) ; 140,8(s) ; 138,4(s) ; 126,2(d) ; 124,5(d) ; 121,9(d) ; 45,6(t) ; 43,9(s) ; 41,6(t) ; 29,6(t) ; 28,6(2q) ; 28,2(t) δ ppm
SM : 202($M^+$,22), 187(100), 159(4), 143(74), 128(40), 115(21), 91(9), 77(6), 63(3), 51(3), 39(4), 29(9)
Odeur : décrite plus haut.

L'acétate de départ a été préparé à partir de 1,1-diméthylindane [5,2 g, 35 mmole ; ce composé est préparé selon des procédés connus ; voir, par exemple, M.T. Bogert et al., J. Amer. Chem. Soc. 56, 185 (1934) ; S.T. Bright et al., J. Org. Chem. 55, 1338 (1990) et réf. y citées] par réaction avec le diacétylacétal d'acroléine (7,9 ml, 52 mmole), en présence de $TiCl_4$ (5,8 ml, 52 mmole) et de $BF_3 \cdot O(C_2H_5)_2$ (0,25 ml, 1 mmole), de façon analogue à la méthode décrite dans l'Exemple 1. Après purification par chromatographie ($SiO_2$, cyclohexane/éther 9:1) et distillation au four à boules (120°/10 Pa), on a obtenu une huile incolore (2,94 g, rend. 32%, pureté 93%) constituée par un mélange d'acétate de (E)-3-(3,3-diméthyl-5-indanyl)-1-propényle (58%) et d'acétate de (E)-3-(1,1-diméthyl-5-indanyl)-1-propényle (25%).
IR(pur) : 2960, 2860, 1755, 1665, 1370, 1225, 1100, 935, 900 $cm^{-1}$
L'isomère majeur susmentionné présentait les caractères analytiques suivants :

RMN($^1$H, 360MHz, $CDCl_3$) : 7,18(dt, $J_1$=12, $J_2$=1,5, 1H) ; 7,10(d, J=8, 1H) ; 6,96(d, J=8, 1H) ; 6,95(s, 1H) ; 5,58(dt, $J_1$=12, $J_2$=7, 1H) ; 3,32(d, J=7, 2H) ; 2,84(t, J=7, 2H) ; 2,11(s, 3H) ; 1,91(t, J=7, 2H) ; 1,25(s, 6H) δ ppm
RMN($^{13}$C, 90,5MHz, $CDCl_3$) : 168,1(s) ; 153,0(s) ; 140,8(s) ; 137,8(s) ; 136,1(d) ; 126,3(d) ; 124,4(d) ; 121,9(d) ; 114,2(d) ; 43,9(s) ; 41,6(t) ; 33,6(t) ; 29,6(t) ; 28,6(2q) ; 20,7(q) δ ppm
SM : 244($M^+$,32), 229(25), 202(21), 187(100), 169(8), 157(7), 146(19), 131(30), 115(30), 91(14), 77(5), 57(9), 43(64)

Exemple 11

Préparation de 3-(1,1-diméthyl-5-indanyl)-2-méthylpropanal et de 3-(3,3-diméthyl-5-indanyl)-2-méthylpropanal

Préparé par hydrolyse de l'acétate de (E)-3-(1,1-diméthyl-5-indanyl)-2-méthyl-1-propényle [2 g, 7 mmole ; mélange 51% de ce composé et 33% de son isomère 3,3-diméthyl-5-indanyl] dans le méthanol (20 ml), à l'aide de $K_2CO_3$ (0,1 g, 0,7 mmole). Après distillation au four à boules (100°/10 Pa), on a obtenu une huile incolore (1,33 g, rend. 84%, pureté 95%) contenant 59% de 3-(1,1-diméthyl-5-indanyl)-2-méthylpropanal et 31% de 3-(3,3-diméthyl-5-indanyl)-2-méthylpropanal.
IR(pur) : 2960, 2860, 2710, 1725, 1490, 1455, 1360, 1125, 835 $cm^{-1}$
Le 3-(1,1-diméthyl-5-indanyl)-2-méthylpropanal présentait les caractères analytiques suivants :

RMN($^1$H, 360MHz, $CDCl_3$) : 9,72(d, J=1,5, 1H) ; 7,04(d, J=7,5, 1H) ; 6,99(s, 1H) ; 6,96(d, J=7,5, 1H) ; 3,04(dd, $J_1$=13, $J_2$=6, 1H) ; 2,84(t, J=7, 2H) ; 2,63(m, 1H) ; 2,57(dd, $J_1$=13, $J_2$=8, 1H) ; 1,91(t, J=7, 2H) ; 1,24(s, 6H) ; 1,09(d, J=7, 3H) δ ppm
RMN($^{13}$C, 90,5MHz, $CDCl_3$) : 204,7(d) ; 150,8(s) ; 143,2(s) ; 136,7(s) ; 127,1(d) ; 125,1(d) ; 121,9(d) ; 48,2(d) ; 43,6(s) ; 41,5(t) ; 36,6(t) ; 30,0(t) ; 28,6(2q) ; 13,3(q) δ ppm
SM : 216($M^+$,18), 201(100), 173(3), 159(15), 143(54), 128(33), 115(17), 91(8), 77(5), 51(3), 39(4), 29(9)
Odeur : décrite plus haut.

L'acétate de départ a été préparé de façon analogue à celle décrite dans l'exemple précédent, à partir de

1,1-diméthylindane (4,75 g, 32 mmole, pureté 98%), à l'aide de $TiCl_4$ (3,5 ml, 32 mmole), $BF_3 \cdot O(C_2H_5)_2$ (0,25 ml, 1 mmole) et diacétylacétal de méthacroléine (5,4 ml, 32 mmole). La distillation au four à boules a fourni une huile incolore (6 g, pureté 65%) contenant 40% d'acétate de (E)-3-(1,1-diméthyl-5-indanyl)-2-méthyl-1-propényle et 22% d'acétate de (E)-3-(3,3-diméthyl-5-indanyl)-2-méthyl-1-propényle.
IR(pur) : 2950, 2855, 1750, 1680, 1480, 1435, 1380, 1365, 1225, 1100, 920, 810 $cm^{-1}$

L'isomère majeur présentait les caractères analytiques suivants :

RMN($^1H$, 360MHz, $CDCl_3$) : 7,05(s, 1H) ; 7,03(d, J=7,5, 1H) ; 7,00(s, 1H) ; 6,98(d, J=7,5, 1H) ; 3,22(s, 2H) ; 2,84 (t, J=7, 2H) ; 2,13(s, 3H) ; 1,91(t, J=7, 2H) ; 1,61(s, 3H) ; 1,23(s, 6H) δ ppm
RMN($^{13}C$, 90,5MHz, $CDCl_3$) : 168,3(s) ; 150,7(s) ; 143,0(s) ; 136,9(s) ; 131,1(d) ; 126,9(d) ; 124,8(d) ; 121,8(d) ; 121,6(s) ; 43,6(s) ; 41,5(t) ; 40,2(t) ; 29,9(t) ; 28,7(2q) ; 20,8(q) ; 13,6(q) δ ppm
SM : 258($M^+$,12), 243(6), 216(27), 201(100), 183(4), 171(3), 159(5), 143(10), 131(37), 115(11), 91(8), 71(5), 43(21)

Exemple 12

Préparation de 6-tert-butyl-1-indaneacétaldéhyde

Préparé de façon analogue à celle décrite dans l'Exemple 4, par oxydation de 2-(6-tert-butyl-1-indanyl)-1-éthanol (8,53 g, 32,1 mmole, pur à 82,3%) dans le dichlorométhane (50 ml), à l'aide de PCC (Fluka, 15,0 g, 68 mmole) dans le dichlorométhane (100 ml). Après le traitement décrit et distillation au four à boules (120°/15 Pa), on a obtenu le composé en titre avec une pureté de 84% (6,01 g, rend. 72,7%). Une purification supplémentaire par chromatographie a fourni le composé désiré pur à 98%, dont les caractères analytiques étaient les suivants :

IR(pur) : 2980, 2880, 2620, 1730, 1495, 1370, 1270, 1125, 830 $cm^{-1}$
RMN($^1H$, 360MHz, $CDCl_3$) : 9,88(s, CHO) ; 7,22(d, J=8, 1H) ; 7,19(s, 1H) ; 7,17(d, J=8, 1H) ; 3,60-3,67(m, 1H) ; 2,80-2,96(m, 3H) ; 2,57-2,66(m, 1H) ; 2,37-2,46(m, 1H) ; 1,65-1,75(m, 1H) ; 1,29(s, 9H) δppm
RMN($^{13}C$, 90,5MHz, $CDCl_3$) : 202,0(d) ; 149,7(s) ; 145,3(s) ; 140,8(s) ; 124,2(d) ; 124,0(d) ; 120,2(d) ; 49,6(t) ; 39,1 (d) ; 34,6(s) ; 32,7(t) ; 31,6(3q) ; 30,9(t) δppm
MS : 216(16), 201(42), 172(45), 159(27), 157(100), 141(21), 131(25), 129(61), 117(33), 115(51), 91(16), 77(6), 57(11)
Odeur : décrite plus haut.

Le 2-(6-tert-butyl-1-indanyl)-1-éthanol de départ est préparé à partir de 3-(4-tert-butyl-1-phényl)propanal, comme suit :

a. 6-tert-butyl-1-indanone
Dissoudre dans un ballon de 500 ml, 20 g du propanal susmentionné pur à 92,2% (96,9 mmole) dans 100 ml d'acétone Fluka puriss, puis ajouter goutte à goutte 55,4 ml de réactif de Jones 2.1 M (116,3 mmole ; 1,2 éq) tout en maintenant la température de la réaction < 30° avec un bain d'eau glacée. Après 16 h à température ambiante, ajouter 10 ml d'isopropanol puis évaporer le solvant. Extraire à l'éther, puis laver avec de la saumure jusqu'à pH neutre et concentrer sous vide. On a obtenu 21,1 g d'acide 3-(4-tert-butylphényl)propionique sous forme de cristaux vert pâle, avec une pureté GC de 90% et les caractères analytiques suivants :

P.f. : 147,5-148°C
IR($CHCl_3$) : 2960 (large), 2640, 1710, 1410, 1270, 835 $cm^{-1}$
RMN($^1H$, 360MHz, $CDCl_3$) : 11,9-11,6(large, COOH) ; 7,32(d, J=8Hz, 2H) ; 7,14(d, J=8Hz, 2H) ; 2,93(t, J=8Hz, 2H) ; 2,68(t, J=8Hz, 2H) ; 1,3(s, 9H) δ ppm
RMN($^{13}C$, 90,5MHz, $CDCl_3$) : 179,6(s) ; 149,2(s) ; 137,1(s) ; 127,9(2d) ; 125,5(2d) ; 35,6(t) ; 34,4(s) ; 31,4 (3q) ; 30,0(t) δ ppm
MS : 206(13), 191(100), 163(5), 145(11), 131(56), 117(27), 91(19), 77(7), 45(7)

Suspendre dans un ballon de 500 ml 21,1 g de l'acide susmentionné (92,1 mmole) dans 10,6 ml de chlorure de thionyle (Fluka purum 99%, 144,7 mmole) puis chauffer le mélange à 60° et laisser agiter à cette température jusqu'à ce que tout l'acide se soit dissous (~ 45 min). Evaporer l'excès de chlorure de thionyle avec le vide de la trompe à eau, puis distiller au four à boules (150°/30 Pa). On a obtenu 12,88 g de chlorure d'acide. Dissoudre dans un ballon de sulfuration de 250 ml 11,88 g de ce chlorure (~ 52,9 mmole) dans 100 ml de $CH_2Cl_2$ puriss, puis refroidir la solution avec un bain de glace et ajouter par petites portions 8,5 g de chlorure d'aluminium (63 mmole). Après 2 h d'agitation à 0°, le mélange réactionnel est versé sur un mélange de glace et d'éther. Le produit est extrait à l'éther, lavé avec de la saumure puis concentré sous vide. On a obtenu 10,3 g de produit brut contenant

86% de 6-tert-butyl-1-indanone et 6,1% de 6-tert-butyl-3-indanone. Le produit a été distillé au four à boules (120°/20 Pa) pour obtenir 8,97g de produit à 95% pur, contenant 89,3% de 6-tert-butyl-1-indanone.

IR($CHCl_3$) : 2960, 1700, 1610, 1490, 1290, 840 $cm^{-1}$
RMN($^1H$, 360MHz, $CDCl_3$) : 7,78(d, J=2Hz, 1H) ; 7,66(dd, J=8, 2Hz, 1H) ; 7,49(d, J=8Hz, 1H) ; 3,09(t, J=6Hz, 2H) ; 2,68(t, J=6Hz, 2H) ; 1,33(s, 9H) δ ppm
RMN($^{13}C$, 90,5MHz, $CDCl_3$) : 207,4(s) ; 152,6(s) ; 150,8(s) ; 137,0(s) ; 132,5(d) ; 126,3(d) ; 120,0(d) ; 36,7(t) ; 34,8(s) ; 31,4(3q) ; 25,33(t) δ ppm
MS : 188(24), 173(100), 145(21), 131(30), 128(9), 115(10), 91(6), 77(4)
Odeur : métallique, poussière, huile à moteur

b. (E)-(6-tert-butyl-1-indanylidène) acétate de méthyle

Dissoudre dans un ballon tricol de 250 ml 8,52 g de la 6-tert-butyl-1-indanone (45,3 mmole) dans 100 ml de pentane. Ajouter ensuite rapidement 10,1 ml de triméthylphosphonoacétate (68 mmole ; 1,5 éq), puis goutte à goutte 11,7 ml de méthylate de sodium (Fluka, 5,4 M dans le méthanol ; 63,4 mmole ; 1,4 éq) et laisser agiter à température ambiante pendant 24 h. Le mélange réactionnel est versé sur un mélange d'éther et de $NaHCO_3$ aqueux saturé, extrait à l'éther, lavé avec de la saumure puis concentré sous vide pour fournir 10,7 g d'un mélange contenant 18,9% de 5-tert-butyl-3(1H)-indèneacétate de méthyle, 4,2% de (Z)-(6-tert-butyl-1-indanylidène) acétate de méthyle et 53,2% de (E)-(6-tert-butyl-1-indanylidène) acétate de méthyle.

Ce produit brut a été utilisé pour l'étape suivante.
IR(pur) : 2980, 1740, 1710, 1680, 1450, 1355, 1200, 1170, 860, 835 $cm^{-1}$
Isomère majoritaire :

RMN($^1H$, 360MHz, $CDCl_3$) : 7,61(d, J=2Hz, 1H) ; 7,42(dd, J=8, 2Hz, 1H) ; 7,28(d, J=8Hz, 1H) ; 6,33(t, J=2Hz, 1H) ; 3,76(s, 3H) ; 3,28-3,34(m, 2H) ; 3,03(t, J=6Hz, 2H) ; 1,34(s, 9H) δ ppm
MS : 244(47), 229(100), 213(19), 197(41), 188(43), 169(17), 155(30), 141(21), 129(38), 115(16), 85(15), 57 (11)

c. 6-tert-butyl-1-indaneacétate de méthyle

L'hydrogénation de 10,7g (76,3% ; 33,4 mmole) du mélange d'esters décrit précédemment s'est effectué en solution dans l'acétate d'éthyle (100 ml) en présence de 2,25 g de Pd 5% sur charbon. La suspension a été agitée pendant 17 h sous atmosphère d'hydrogène et a donné, après filtration du catalyseur et concentration, 10,7 g de 6-tert-butyl-1-indaneacétate de méthyle brut avec une pureté de 80,5%.

Le produit brut a été utilisé pour l'étape suivante.

IR(pur) : 2990, 2940, 1740, 1500, 1440, 1370, 1270, 1180, 830 $cm^{-1}$
RMN($^1H$, 360MHz, $CDCl_3$) : 7,14-7,23(m, 3H) ; 3,72(s, 3H) ; 3,53-3,62(m, 1H) ; 2,76-2,94(m, 3H) ; 2,33-2,48 (m, 2H) ; 2,70-2,80(m, 1H) ; 1,3(s, 9H) δ ppm
RMN($^{13}C$, 90,5MHz, $CDCl_3$) : 173,31(s) ; 149,48(s) ; 145,45(s) ; 140,88(s) ; 124,08(d) ; 123,94(d) ; 120,25(d) ; 51,53(q) ; 41,51(d) ; 39,85(t) ; 34,60(s) ; 32,62(t) ; 31,58(q) ; 30,67(t) δ ppm
MS : 246(27), 231(59), 189(41), 173(31), 157(100), 129(70), 115(29), 91(8), 57(15)

d. 2-(6-tert-butyl-1-indanyl)-1-éthanol

A une suspension de 1,72 g de $LiAlH_4$ (45,03 mmole) dans 100 ml d'éther a été additionnée goutte à goutte une solution de 10,7 g de 6-tert-butyl-1-indanacétate de méthyle (36,9 mmole) dissous dans 50 ml d'éther. Après avoir laissé refluer la suspension pendant 2 h, 8,6 ml de NaOH 1 N ont été prudemment ajoutés et, après 1 h, la suspension a été filtrée et concentrée pour donner 9,05 g de 2-(6-tert-butyl-1-indanyl)-1-éthanol brut pur à 78,1%.

La distillation au four à boules (150°/20 Pa) a fourni 8,53 g de 2-(6-tert-butyl-1-indanyl)-1-éthanol avec une pureté GC de 82,3%. Rendement pour les trois dernières étapes : 71,0%.

IR(pur) : 3320, 2960, 2860, 1480, 1355, 1255, 1150, 820 $cm^{-1}$
RMN($^1H$, 360MHz, $CDCl_3$) : 7,24(d, J=1Hz, 1H) ; 7,21(dd, J=8, 1, 1H) ; 7,15(d, J=8Hz, 1H) ; 3,76-3,84(m, 2H) ; 3,17-3,27(m, 1H) ; 2,75-2,94(m, 2H) ; 2,26-2,36(m, 1H) ; 2,12-2,21(m, 1H) ; 1,64-1,76(m, 2H) ; 1,40-1,44(large, OH) ; 1,32(s, 9H) δ ppm
RMN($^{13}C$, 90,5MHz, $CDCl_3$) : 149,3(s) ; 146,8(s) ; 140,9(s) ; 124,0(d) ; 123,5(d) ; 120,4(d) ; 61,6(t) ; 41,6(d) ; 38,0(t) ; 34,6(s) ; 32,5(t) ; 31,6(3q) ; 30,9(t) δ ppm
MS : 218(60), 203(100), 185(29), 173(84), 161(84), 157(52), 143(84), 129(60), 117(70), 91(22), 77(10), 57(50)

Exemple 13

Préparation de 3-(5-indanyl)-1-propanal et de 3-(4-indanyl)-1-propanal

Préparé par hydrolyse d'acétate de (E)-3-(5-indanyl)-1-propényle [3,3 g ; 13,6 mmole ; mélange pur à 89%, contenant 74% de ce composé et 15% d'acétate de (E)-3-(4-indanyl)-1-propényle], à l'aide d'acide sulfurique à 25% (6 ml) dans le THF (25 ml), de façon analogue à celle décrite dans l'Exemple 1. Après 4 h de reflux et le traitement décrit, on a distillé au four à boules (125°/10 Pa) pour obtenir 2,07 g d'un produit ayant une pureté de 99% et contenant 83% de 3-(5-indanyl)-1-propanal et 16% de 3-(4-indanyl)-1-propanal (rend. 87%).
Caractères analytiques :
IR(pur) : 2950, 2840, 2710, 1720, 1490, 1435, 1060, 820 $cm^{-1}$
Isomère majoritaire :

RMN($^1H$, 360MHz, $CDCl_3$) : 9,78(d, J=1,5Hz, CHO) ; 7,13(d, J=8Hz, 1H) ; 7,05(s, 1H) ; 6,94(d, J=8Hz, 1H) ; 2,91 (t, J=8Hz, 2H) ; 2,87(t, J=7Hz, 2H) ; 2,86(t, J=7Hz, 2H) ; 2,73(dt, J=1,5, 8Hz, 2H) ; 2,05(quint, J=7Hz, 2H) $\delta$ ppm
RMN($^{13}C$, 90,5MHz, $CDCl_3$) : 201,8(s) ; 144,7(s) ; 142,2(s) ; 138,1(s) ; 126,1(d) ; 124,4(d) ; 124,3(d) ; 45,6(t) ; 32,8 (t) ; 32,4(t) ; 28,0(t) ; 25,5(t) $\delta$ ppm
MS : 174(73), 156(3), 145(14), 131(76), 128(33), 118(100), 103(7), 91(37), 77(12), 65(8), 63(9), 51(9), 39(10), 29 (14)
Odeur : décrite plus haut.

L'acétate de départ est préparé ainsi :

Dissoudre dans un ballon tricol de 200 ml, 6,4 g d'indane (Fluka purum 95%, 50 mmole) dans 50 ml de $CH_2Cl_2$ puriss, puis ajouter goutte à goutte rapidement à température ambiante 6,0 ml de $TiCl_4$ (Fluka, 55 mmole ; 1,1 éq) puis 0,12 ml de $BF_3.Et_2O$ (Fluka 48%, 1 mmole ; 0,02 éq). Après avoir laissé agiter à température ambiante pendant 30 min, la solution est refroidie à -10°, puis une solution de 7,9 g d'acroleine diacétyl acétal (Fluka 98%, 50 mmole) dissous dans 20 ml de $CH_2Cl_2$ est ajoutée goutte à goutte en 30 min. Après avoir laissé agiter la solution pendant 1 h à -5°, le mélange réactionnel est décomposé sur un mélange de glace et HCl à 10%, puis extrait à l'éther. La phase éthérée est ensuite lavée avec de la saumure jusqu'à pH neutre, puis séchée sur $Na_2SO_4$ et concentrée sous vide. On a obtenu 10,9 g d'un liquide brun qui a été distillé au four à boules (185°/10 Pa) pour fournir 4,51 g d'un produit contenant 74,0% d'acétate de (E)-3-(5-indanyl)-1-propényle et 15,0% d'acétate de (E)-3-(4-indanyl)-1-propényle (rend. 37,0%).
IR(pur) : 2960, 2840, 1755, 1670, 1430, 1370, 1225, 1100, 940, 900 $cm^{-1}$
Les données suivantes sont pour l'isomère majoritaire :

RMN($^1H$, 360MHz, $CDCl_3$) : 7,17(dt, J=12, 1Hz, 1H) ; 7,13(d, J=7Hz, 1H) ; 7,05(s, 1H) ; 6,95(d, J=7Hz, 1H) ; 5,57 (dt, J=12, 8Hz, 1H) ; 3,28(d, J=8Hz, 2H) ; 2,86(t, J=7Hz, 4H) ; 2,1(s, 3H) ; 2,05(m, 2H) $\delta$ ppm
RMN($^{13}C$, 90,5MHz, $CDCl_3$) : 168,2(s) ; 144,6(s) ; 142,2(s) ; 137,5(s) ; 136,1(d) ; 126,2(d) ; 124,3(2d) ; 114,2(d) ; 33,4(t) ; 32,8(t) ; 32,5(t) ; 25,5(t) ; 20,7(q) $\delta$ ppm
MS : 216(47), 174(65), 156(15), 145(28), 131(44), 118(89), 91(22), 77(7), 63(7), 55(7), 43(100), 39(7)

Exemple 14

Préparation de 3-(5-indanyl)-2-méthyl-1-propanal

Préparé par hydrolyse d'acétate de (E)-3-(5-indanyl)-2-méthyl-1-propényle [4 g, 17,4 mmole; mélange pur à 96%, contenant 84,1% de ce composé et 11,7% d'acétate de (E)-3-(4-indanyl)-2-méthyl-1-propenyle], à l'aide de 10 ml d'acide sulfurique à 25% et dans 50 ml de THF, de façon analogue à celle décrite dans l'exemple précédent. Après traitement et distillation au four à boules (110°/15 Pa) on a obtenu 2,81 g d'un produit à 96% pur, contenant 3-(5-indanyl)-2-méthyl-1-propanal et 3-(4-indanyl)-2-méthyl-1-propanal dans un rapport 9:1 (rend. 82,4%).
Caractères analytiques :
IR(pur) : 2940, 2860, 2700, 1750, 1500, 1450, 915, 835, 800 $cm^{-1}$
Isomère majoritaire :

RMN($^1H$, 360MHz, $CDCl_3$) : 9,71(d, J=2Hz, CHO) ; 7,13(d, J=8Hz, 1H) ; 7,03(s, 1H) ; 6,93(d, J=8Hz, 1H) ; 3,03 (dd, J=13, 5Hz, 1H) ; 2,86(t, J=7,5Hz, 4H) ; 2,63(m, 1H) ; 2,57(dd, J=13, 8Hz, 1H) ; 2,05(quint, J=7,5Hz, 2H) ; 1,08 (d, J=8Hz, 3H) $\delta$ ppm
RMN($^{13}C$, 90,5MHz, $CDCl_3$) : 204,6(d) ; 144,6(s) ; 142,3(s) ; 136,5(s) ; 126,8(d) ; 125,0(d) ; 124,3(d) ; 48,3(d) ;

36,6(t) ; 32,8(t) ; 32,5(t) ; 25,5(t) ; 13,3(q) δ ppm
MS : 188(31), 173(2), 160(4), 145(5), 131(100), 128(15), 118(28), 115(24), 91(20), 77(6), 29(8)
Odeur : hespéridée, métallique, citronellal.

L'acétate de départ est préparé ainsi :

Dissoudre dans un ballon tricol de 200 ml, 6,5 ml d'indane (Fluka purum 95%, 50 mmole) dans 40 ml de $CH_2Cl_2$ puriss puis ajouter goutte à goutte rapidement à température ambiante 6,0 ml de $TiCl_4$ (Fluka, 55 mmole ; 1,1 éq) puis 0,39 ml de $BF_3.Et_2O$ (Fluka 48%, 1,5 mmole ; 0,03 éq). Après avoir laissé agiter à température ambiante pendant 30 min, la solution est refroidie à -10°, puis une solution de 9,22 ml de méthacroleine diacétyl acétal (Fluka 98%, 50 mmole) dissous dans 25 ml de $CH_2Cl_2$ est ajoutée goutte à goutte en 30 min. Après avoir laissé agiter la solution pendant 1 h à -5°, le mélange réactionnel est décomposé sur un mélange de glace et HCl à 10%, puis extrait à l'éther. La phase éthérée est ensuite lavée avec de la saumure jusqu'à pH neutre, puis séchée sur $Na_2SO_4$ est concentrée sous vide. On a obtenu 11,2 g d'un liquide brun qui a été distillé au four à boules (140°/15 Pa) pour fournir 4,18 g d'un produit contenant 84,1% d'acétate de (E)-3-(5-indanyl)-2-méthyl-1-propényle et 11,7% d'acétate de (E)-3-(4-indanyl)-2-méthyl-1-propényle (rend. 34,7%).
IR(pur) : 2942, 1752, 1490, 1369, 1228, 1099, 921, 813 $cm^{-1}$
Isomère majoritaire :

RMN($^1H$, 360MHz, $CDCl_3$) : 7,13(d, J=8Hz, 1H) ; 7,04(s, 2H) ; 6,94(d, J=8Hz, 1H) ; 3,21(s, 2H) ; 2,86(t, J=7,5Hz, 4H) ; 2,13(s, 3H) ; 2,05(quint, J=7,5Hz, 2H) ; 1,6(d, J=2Hz, 3H) δ ppm
RMN($^{13}C$, 90,5MHz, $CDCl_3$) : 168,3(s) ; 144,5(s) ; 142,2(s) ; 136,7(s) ; 131,1(d) ; 126,6(d) ; 124,7(d) ; 124,2(d) ; 121,5(s) ; 40,2(t) ; 32,8(t) ; 32,5(t) ; 25,5(t) ; 20,8(q) ; 13,6(q) δ ppm
MS : 230(32), 188(53), 170(10), 160(10), 155(7), 145(11), 131(73), 118(100), 91(21), 77(6), 43(59)

Exemple 15

Préparation d'une composition parfumante

On a préparé une composition parfumante de base destinée à un parfum de type féminin en mélangeant les ingrédients suivants :

| Ingrédients | Parties en poids |
|---|---|
| Acétate de benzyle | 15 |
| Acétate de géranzyle | 8 |
| Acétate de linalyle | 35 |
| Acétate de styrallyle | 4 |
| Alcool cinnamique à 10%* | 6 |
| Aldéhyde anisique à 10%* | 5 |
| Cyclosia® Base 1) | 7 |
| Damascénone à 10%* | 15 |
| Dorinone® Bêta 2) à 10% dans le citrate d'éthyle | 12 |
| Ethyle linalol | 20 |
| Eugénol | 25 |
| Exaltolide® 3) | 17 |
| Galaxolide® 4) 50 | 55 |
| Hédione® 5) | 60 |
| Héliotropine | 44 |
| Hexylix® 6) à 10% * | 20 |

* dans le dipropylèneglycol (DIPG)
1) mélange à base d'hydroxycitronellal ; origine : Firmenich SA, Genève, Suisse
2) 1-(2,6,6-triméthyl-1-cyclohexén-1-yl)-2-butén-1-one ; origine : Firmenich SA, Genève, Suisse
3) pentadécanolide ; origine : Firmenich SA, Genève, Suisse
4) 1,3,4,6,7,8-hexahydro-4,6,6,7,8,8-hexaméthyl-cyclopenta-γ-2-benzopyrane ; origine : International Flavors & Fragrances Inc., USA
5) dihydrojasmonate de méthyle ; origine : Firmenich SA, Genève, Suisse
6) acétate d'allyl (cyclohexyloxy) ; origine : Charabot, France

(suite)

| Ingrédients | Parties en poids |
|---|---|
| Indol à 10% dans la triéthylamine | 32 |
| Iso E Super 7) | 100 |
| Levocitrol | 24 |
| Linalol | 20 |
| Phénéthylol | 5 |
| Polysantol® 8) à 10%* | 60 |
| Polywood® 9) Super | 15 |
| Salicylate de benzyle | 110 |
| Salicylate de pipol | 30 |
| Essence de tagète à 10%* | 12 |
| α-Terpinéol | 45 |
| Vanilline à 10%* | 8 |
| α-Ionone | 14 |
| β-Ionone | 52 |
| Dianthine® 10) SA | 5 |
| Total | 880 |

* dans le dipropylèneglycol (DIPG)

7) 1-octahydro-2,3,8,8-tétraméthyl-2-naphtalényl)-1-éthanone ; origine : International Flavors & Fragrances Inc., USA

8) 3,3-diméthyl-5-(2,2,3-triméthyl-3-cyclopentén-1-yl)-4-pentén-2-ol ; origine : Firmenich SA, Genève, Suisse

9) acétate de perhydro-5,5,8aα-triméthyl-2α-trans-naphtalényle ; origine : Firmenich SA, Genève, Suisse

10) origine : Firmenich SA, Genève, Suisse

A cette composition de base de type florale, verte, on a ajouté d'une part 120 parties en poids de 3-(5-tert-butyl-2-méthyl-1-phényl)propanal selon l'invention pour préparer une composition nouvelle A et, d'autre part, 120 parties en poids de 3-(4-tert-butyl-1-phényl)-2-méthylpropanal, ou LILIAL®, pour préparer une composition B.

Les deux compositions ont ensuite été évaluées à l'aveugle par un panel de 13 experts parfumeurs. De l'avis unanime de ces derniers, la composition nouvelle A a été préférée pour sa note florale beaucoup plus douce et naturelle que celle de la composition B. Les parfumeurs ont aussi trouvé que l'odeur de la composition A était plus puissante et avait plus de volume, la note odorante apparaissant bien plus poudrée et les caractères jasminé et muguet étant nettement exaltés.

Lorsqu'on a ajouté à la composition de base 120 parties en poids de 2-(5-tert-butyl-2-méthylbenzyl)propanal selon l'invention, on a obtenu une nouvelle composition C dont l'odeur était très proche de celle de la composition A, la note florale étant cependant accompagnée d'un caractère plus vert.

Exemple 16

Préparation d'une composition parfumante

On a préparé une composition parfumante de base, destinée à un détergent en poudre, par mélange des ingrédients suivants :

| Ingrédients | Parties en poids |
|---|---|
| Acétate de carbinol | 15 |
| Acétate de linalyle | 30 |
| (3 et 4)-(4-méthyl-3-pentén-1-yl)-3-cyclohexène-3-carbaldéhyde | 20 |
| Aldéhyde amylcinnamique | 125 |
| Aldéhyde undécylénique à 50%* | 15 |
| Aldéhyde méthyl nonylique à 50%* | 15 |
| Citronellol | 15 |

* dans le DIPG

(suite)

| Ingrédients | Parties en poids |
|---|---|
| Dihydromyrcenol® 1) | 15 |
| Exaltolide® 2) à 10%* | 30 |
| Géraniol brut | 30 |
| Héliotropine ordinaire | 15 |
| Iralia® 3) | 90 |
| Linalol | 25 |
| Lorysia® 4) | 110 |
| Méthylanthranilate de méthyle | 5 |
| Essence de Patchouli | 30 |
| Phénylhexanol | 25 |
| Polysantol® 5) | 20 |
| Polywood® 6) Super | 10 |
| Spiranol 7) | 10 |
| Terpinéol | 50 |
| Tonalid® 8) | 70 |
| Vert de lilas | 10 |
| Vertofix coeur 9) | 40 |
| Dorinia SA 10) | 20 |
| Galbex® 11)183 | 10 |
| Total | 850 |

* dans le DIPG

1) 2,6-diméthyl-7-octén-2-ol ; origine : International Flavors & Fragrances Inc., USA

2) voir exemple 3

3) méthylionone (mélange isomérique) ; origine : Firmenich SA, Genève, Suisse

4) acétate de 4-(1,1-diméthyléthyl)-1-cyclohexyle ; origine : Firmenich SA, Genève, Suisse

5) voir exemple 3

6) voir exemple 3

7) 2,6,10,10-tétraméthyl-1-oxaspiro[4.5]décan-6-ol ; origine : Firmenich SA, Genève, Suisse

8) (5,6,7,8-tétrahydro-3,5,5,6,8,8-hexaméthyl-2-hexaméthyl-2-naphtyl)-1-éthanone ; origine : PFW, Hollande

9) origine : International Flavors & Fragrances Inc., USA

10) origine : Firmenich SA, Genève, Suisse

11) origine : Firmenich SA, Genève, Suisse

A cette composition de base de type floral ont été ajoutées 150 parties en poids de 5-tert-butyl-2-indanecarbaldéhyde pour préparer une nouvelle composition A et 150 parties en poids de 3-(4-tert-butyl-1-phényl)propanal, ou BOURGEONAL®, pour préparer une composition B.

Ces deux compositions ont ensuite été utilisées à concentration identique pour préparer deux échantillons, respectivement A et B d'un détergent en poudre parfumé.

Un panel de 7 parfumeurs, évaluant ces deux échantillons de détergent à l'aveugle a montré une nette préférence pour l'échantillon A dont l'odeur a été jugée plus puissante et plus élégante que celle de l'échantillon B.

Deux lots standards de textiles ont ensuite été lavés séparément dans deux machines avec les échantillons A et B et l'odeur des textiles a été évaluée à l'aveugle par un panel de 6 experts parfumeurs. L'évaluation a eu lieu sur des textiles humides, à la sortie de la machine, ainsi qu'après 24 h de séchage à l'air.

Les parfumeurs ont unanimement préféré l'odeur des textiles traités avec l'échantillon A, aussi bien à l'état humide qu'après séchage. L'odeur de ce lot de textiles a été trouvée nettement supérieure, du point de vue de la puissance et qualité, à celle des textiles lavés avec l'échantillon B.

L'odeur des linges humides lavés avec l'échantillon A a été jugée beaucoup plus fleurie que celle des textiles traités avec l'échantillon B, alors que les textiles secs exhalaient une odeur beaucoup plus puissante, odeur qui restait aussi sur les textiles beaucoup plus longtemps que celle des textiles traités avec l'échantillon B.

Exemple 17

Test de stabilité sur mouillette

On a effectué des tests de stabilité sur mouillette, en comparant la performance de certains composés de l'invention, à savoir le 3-(5-tert-butyl-2-méthyl-1-phényl)propanal (mouillette A), le 3-(5-tert-butyl-2-méthylbenzyl) propanal (mouillette B), le 5-tert-butyl-2-indanecarbaldéhyde (mouillette C), et le 3-(3,3-diméthyl-5-indanyl)-1-propanal (mouillette F), avec celle de leurs deux analogues connus, à savoir le 3-(4-tert-butyl-1-phényl)propanal ou BOURGEONAL® (mouillette D) et le 3-(4-tert-butyl-1-phényl)-2-méthylpropanal ou LILIAL® (mouillette E).

Pour ce faire, un panel de 4 experts parfumeurs a trempé des mouillettes dans des flacons contenant les composés susmentionnés à l'état pur, de façon à obtenir une zone imprégnée d'environ 1 cm dans chaque cas. Ces mouillettes ont ensuite été évaluées à l'aveugle, et leurs odeurs comparées, dans le temps, cette opération étant répétée tous les jours jusqu'à ce que les parfumeurs ne décèlent plus d'odeur sur aucune des mouillettes.

De l'avis des parfumeurs, au départ, les mouillettes A et B développaient des odeurs florales où la connotation de type muguet était nettement dominante.

La mouillette A exhalait en plus une note rappelant l'odeur du thym. La mouillette C, pour sa part, développait une odeur florale remarquablement puissante, avec un caractère plus vert et plus proche de l'odeur du BOURGEONAL® (mouillette D).

La mouillette D avait une odeur florale, beaucoup plus verte et aldéhydée que celle des mouillettes A et B, plus agressive aussi, et la mouillette E possédait une odeur florale du même type de celle des mouillettes A et B, mais dont le caractère muguet était beaucoup moins marqué.

Finalement, la mouillette F développait une odeur florale où la connotation verte était très puissante, rappelant l'odeur que l'on associe au linge fraîchement lavé.

L'évolution de l'intensité de l'odeur des six mouillettes dans le temps, telle que jugée par les parfumeurs sur une échelle de valeurs allant de 0 à 10, est indiquée dans le tableau suivant :

| Mouillette | 3 jours | 7j | 9j | 12j | 15j | 20j |
|---|---|---|---|---|---|---|
| A | 7 | 3 | - | - | - | - |
| B | 6 | - | - | - | - | - |
| C | 5 | 5 | 5 | 4 | 4 | 3 |
| D | 8 | 4 | - | 1 | - | - |
| E | - | - | - | - | - | - |
| F | 7 | - | 6 | 4 | 4 | 3 |

Ainsi, il a été constaté que les mouillettes A et B, qui développaient au départ des odeurs proches de celle de la mouillette E et, du moins, tout aussi puissantes, conservaient cette odeur pendant environ 5 jours, alors que l'odeur de la mouillette E diminuait fortement d'intensité dans les premières 24 h et n'était plus détectable à la fin de 3 jours.

D'autre part, la mouillette D maintenait une odeur intense à la fin de 3 jours, qui s'estompait cependant rapidement dans la semaine qui suivait, alors que la mouillette C, dont l'intensité de l'odeur était au départ inférieure à celle de la mouillette D, mais semblable, conservait par la suite une intensité pratiquement stable et exhalait encore une fragrance parfaitement perceptible 20 jours après avoir été trempée dans le 5-tert-butyl-2-indanecarbaldéhyde selon l'invention. Par ailleurs, de l'avis des parfumeurs, la qualité de l'odeur de la mouillette C n'avait souffert d'aucune altération à la fin de cette période. Un comportement similaire avait été observé avec la mouillette F qui avait conservé son odeur pendant plus d'un mois, révélant ainsi la tenacité remarquable du 3-(3,3-diméthyl-5-indanyl)-1-propanal selon l'invention.

Exemple 18

Test de stabilité à l'oxydation par chromatographie en phase gazeuse (GC)

L'évolution qualitative décrite dans l'exemple précédent pour le 3-(5-tert-butyl-2-méthyl-1-phényl)propanal et ses analogues connus, sur la base de l'évaluation odorante des parfumeurs, a été aussi constatée pour ces composés, de façon quantitative, à l'aide de mesures par chromatographie en phase gazeuse (GC).

On a procédé comme suit.

On a déposé sur des mouillettes standard (7x147 mm) une goutte de, respectivement, 3-(5-tert-butyl-2-méthyl-1-phényl)propanal (mouillette A), 3-(4-tert-butyl-1-phényl)propanal ou BOURGEONAL® (mouillette B) et de 3-(4-tert-butyl-1-phényl)-2-méthylpropanal ou LILIAL® (mouillette C).

La zone des mouillettes ainsi imprégnée (~ 20 mm) a été coupée et plongée pendant 1h dans du $CH_2Cl_2$ (1 ml) contenu dans des tubes à essai fermés, en agitant occasionnellement.

Avant d'injecter les solutions dans un appareil GC, on a ajouté de la bis-(triméthylsilyl)-acétamide (Aldrich, 4 gouttes, ~30 mg) à chacune des trois solutions, pour former l'ester triméthylsilylique correspondant à l'acide formé par oxydation à l'air de l'aldéhyde extrait de chacune des mouillettes. Il a, en effet, été constaté que le signal GC desdits esters était nettement moins large que celui de l'acide correspondant, et permettait donc une intégration beaucoup plus précise.

Les trois solutions ont ensuite été injectées dans un appareil GC (colonne de $SiO_2$, 10 m) à périodes régulières, adaptées à la vitesse d'oxydation observée pour chacun des trois aldéhydes susmentionnés. Les signaux correspondant à l'aldéhyde et à l'ester triméthylsilylique (proportionnel à la quantité d'acide formé) on été intégrés et les résultats obtenus représentés sur le graphique à la Figure 1.

Sur ce graphique, le pourcentage d'aldéhyde et d'acide correspondant sont représentés en fonction du temps. Les courbes représentées traduisent les valeurs moyennes obtenues pour deux expériences distinctes, effectuées avec chacun des composés dont les structures sont représentées.

Il résulte clairement de la Figure 1 que le composé selon l'invention, à savoir le 3-(5-tert-butyl-2-méthyl-1-phényl)propanal, est beaucoup plus stable à l'oxydation à l'air que son isomère connu, le 3-(4-tert-butyl-1-phényl)-2-méthylpropanal ou LILIAL®, lequel, au bout d'environ 4 jours, s'est transformé à 80% en l'acide correspondant, pratiquement inodore.

Lorsque l'on compare le composé de l'invention à son homologue inférieur connu, à savoir le 3-(4-tert-butyl-1-phényl)propanal ou BOURGEONAL® (origine : Naarden Intl, Hollande), on voit de nouveau clairement que ce dernier, bien que plus stable que le LILIAL®, à la fin d'une vingtaine de jours, s'est transformé à 70% en l'acide correspondant, alors que l'aldéhyde selon la présente invention reste à ~60% stable.

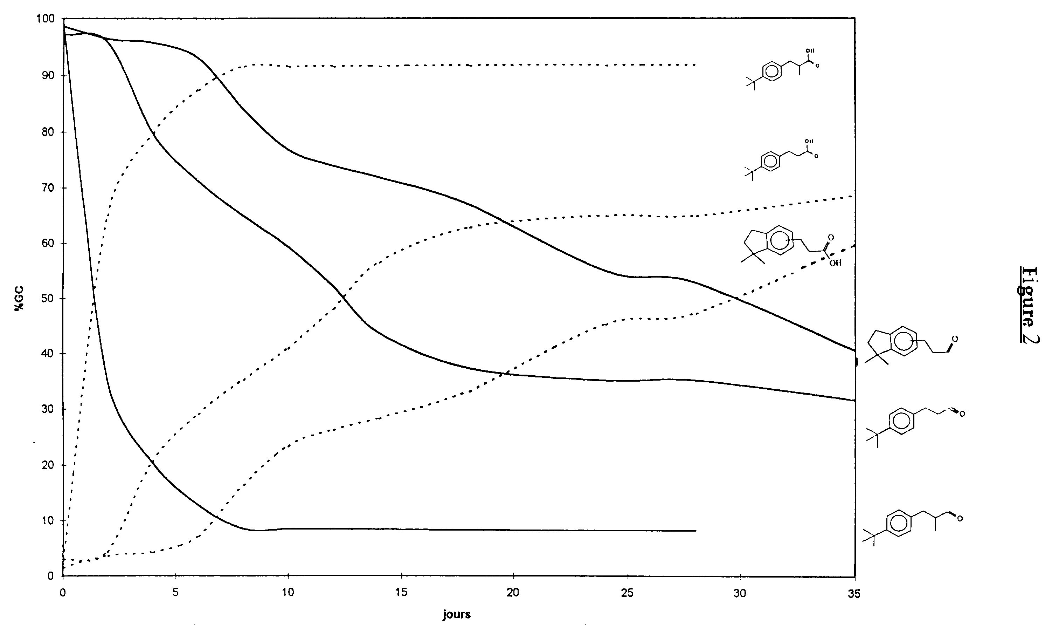

Des expériences analogues ont été effectuées avec un autre composé selon l'invention, à savoir le 3-(3,3-diméthyl-5-indanyl)propanal, et les résultats, représentés à la Figure 2, montrent également que ce composé est plus stable que les deux composés connus cités plus haut.

Il convient de noter que ces résultats ne pouvaient être attribués à des différences de volatilité et/ou polarité des composés de l'invention par rapport à leurs analogues connus, le LILIAL® et le BOURGEONAL®. Nous avons, en effet, mesuré les temps de rétention de ces deux composés sur deux types de colonnes GC (100-20°, 10°/min) et les résultats présentés ci-après montrent qu'il n'y a pas de différences significatives dans ces valeurs.

| Temps rétention GC [min] | LILIAL® | BOURGEONAL® | 3-(5-tert-butyl-2-méthyl-1-phényl) propanal | 3-(3,3-diméthyl-5-indanyl)propanal (2 isomères) |
|---|---|---|---|---|
| Colone silice (10 m) | 4,01 | 4,10 | 4,34 | 5,03 ; 5,17 |
| Colone Carbowax (10 m) | 5,62 | 5,69 | 6,78 | 6,46 ; 6,80 |

<u>Exemple 19</u>

<u>Base parfumante pour détergent en poudre</u>

On a préparé une base parfumante concentrée en mélangeant les ingrédients suivants :

| Ingrédients | Parties en poids |
|---|---|
| Acétate de benzyle | 200 |
| Acétate de linalyle | 70 |
| Acétate de verdyle | 160 |
| Aldéhyde anisique | 60 |
| Décanal 10%* | 10 |
| Aldéhyde hexylcinnamique | 300 |
| Aldéhyde méthylnonylique à 10%* | 10 |

* dans le dipropylèneglycol

(suite)

| Ingrédients | Parties en poids |
|---|---|
| Anthranilate de méthyle | 25 |
| Astrotone 1) | 150 |
| Coranol 2) 3) | 50 |
| Cyclohexylpropionate d'allyle | 10 |
| Ethylvanilline 10%* | 70 |
| Hédione ® 3) 4) | 100 |
| Héliotropine | 25 |
| Galaxolide 50 5) 6) | 380 |
| Iralia ® Total 3) 7) | 600 |
| Iso E Super 5) 8) | 250 |
| Koavone 5) 9) | 70 |
| Méthylnaphtylcétone | 10 |
| Acétate de p-tert-butylcyclohexanone | 250 |
| Phénylhexanol | 30 |
| Propionate de verdyle | 100 |
| Salicylate d'amyle | 60 |
| Galbex ® 183 3) | 10 |
| Total | 3000 |

* dans le dipropylèneglycol
1) éthylène undécane dicarboxylate
2) 4-cyclohexyl-2-méthyl-2-butanol
3) origine : Firmenich SA, Genève, Suisse
4) 3-oxo-2-pentyl-cyclopentylacétate de méthyle
5) origine : International Flavors & Fragrances Inc., USA
6) 1,3,4,6,7,8-hexahydro-4,6,6,7,8,8-hexaméthyl-cyclopenta-γ-2-benzopyrane
7) iso-méthyl-ionone
8) 7-acétyl-1,2,3,4,5,6,7,8-octahydro-1,1,6,7-tétraméthyl-naphtalène
9) acétyl-diisoamylène

Lorsqu'on ajoute à 30 parties en poids de la composition ainsi obtenue 3 parties en poids de 3-(3,3-diméthyl-5-indanyl)-1-propanal, on obtient une nouvelle composition dont la note verte est nettement renforcée. La composition acquiert par ailleurs un caractère frais très marqué. Une telle note olfactive était tout aussi bien développé dans une poudre de détergent que sur un linge humide après lavage avec le détergent parfumé à l'aide de ladite composition.

Une fois séché, le linge (après 24 h) était caractérisé par une odeur aldéhydée, verte, fraîche qui éveillait instantanément chez l'utilisateur une agréable sensation de linge très propre.

Lorsqu'on a ajouté à la composition de base la même proportion de 5-tert-butyl-2-indanecarbaldéhyde on a obtenu un effet olfactif semblable à celui décrit ci-dessus mais un peu moins puissant, alors que l'addition de la même quantité de 6-tert-butyl-1-indaneacétaldéhyde conférait à la composition une odeur de type vert-feuillage plus prononcée mais où le caractère aldéhydémuguet n'était pas aussi perceptible que dans le cas de l'addition des deux composés cités ci-dessus.

Exemple 20

Base parfumante

On a préparé une composition de base de type floral, boisé, destinée à un savon, en mélangeant les ingrédients suivants :

| Ingrédients | Parties en poids |
|---|---|
| Acétate de benzyle | 250 |
| Acétate de citronellyle | 150 |
| Acétate de phényléthyle | 120 |

(suite)

| Ingrédients | Parties en poids |
|---|---|
| Acétate de styrallyle | 100 |
| Aldéhyde anisique | 100 |
| Aldéhyde undécylénique à 50%* | 120 |
| Aldéhyde hexylcinnamique | 200 |
| 4-(4-hydroxy-1-phényl)-2-butanone | 5 |
| Citronellol | 150 |
| Dihydromyrcenol [1) 5)] | 200 |
| Ethylvanilline à 10%* | 30 |
| Eugénol | 60 |
| Lilial® [2)] | 120 |
| Hédione® [3) 4)] | 100 |
| Méthyl-p-crésol à 10%* | 40 |
| Vertofix coeur [5)] | 430 |
| Iralia® Total [3) 6)] | 130 |
| γ-Undécalactone | 5 |
| Phénéthylol | 300 |
| Phénylacétate de phénylhexyle | 20 |
| Acétate de p-tert-butylcyclohexanone | 750 |
| Polysantol® [1) 3)] | 20 |
| Propionate de verdyle | 250 |
| Salicylate d'hexyle | 400 |
| Tonalid® [1)] | 100 |
| Essence de violette | 20 |
| Ylang synthétique | 50 |
| Total | 4220 |

* dans le dipropylèneglycol
1) voir exemple 16
2) origine : Givaudan-Roure, Vernier, Suisse
3) origine : Firmenich SA, Genève, Suisse
4) 3-oxo-2-pentyl-cyclopentylacétate de méthyle
5) origine : International Flavors & Fragrances Inc., USA
6) iso-méthyl-ionone

L'addition, à cette composition de base, de 80 parties en poids de 6-tert-butyl-1-indaneacétaldéhyde lui a conféré une note aldéhydée-verte qui rappelait l'effet olfactif que l'on peut obtenir avec le BOURGEONAL®, alors que lorsqu'on ajoutait la même quantité de 5-tert-butyl-2-indanecarbaldéhyde ou de 3-(3,3-diméthyl-5-indanyl)-1-propanal, on obtenait un effet floral-muguet nettement distinct et d'autant plus puissant dans le dernier cas. Ce dernier composé impartissait également, de façon plus marquée, un agréable caractère frais, linge propre à la composition de base.

## Revendications

**1.** Composé de formule

a)

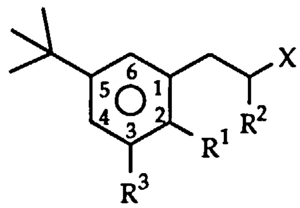

(Ia)

dans laquelle le symbole X représente un groupe - CHO ou un groupe

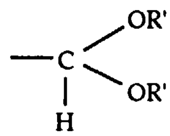

les symboles R', pris isolément, représentant chacun un radical alkyle de $C_1$ à $C_4$, linéaire ou ramifié, saturé ou insaturé, ou, pris ensemble, représentant un radical alkylène de $C_2$ à $C_4$, éventuellement substitué ; le symbole $R^2$ représente un atome d'hydrogène ou un radical méthyle ; et $R^1$ et $R^3$ sont différents et représentent chacun un atome d'hydrogène ou un radical méthyle, le 2-(5-tert-butyl-2-méthylbenzyl)propanal étant exclu ;
ou de formule
b)

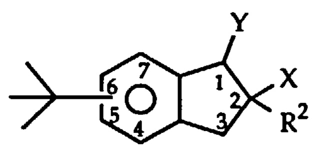

(Ib)

dans laquelle le radical tert-butyle se trouve en position 5 ou 6 du cycle aromatique et soit Y représente l'hydrogène et X et $R^2$ ont le sens indiqué ci-dessus, soit X et $R^2$ représentent chacun un atome d'hydrogène et Y représente un groupe - $CH_2CHO$ ou un groupe

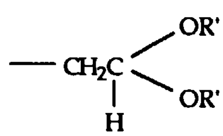

où R' est défini comme en a);
ou de formule
c)

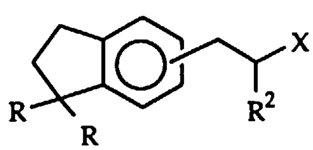

(Ic)

dans laquelle X et $R^2$ ont le sens indiqué à la formule (Ia) et R représente un atome d'hydrogène ou un radical méthyle, les groupes R pouvant être identiques ou différents.

**2.** A titre de composé selon la revendication 1, l'un des composés suivants :

a. 3-(5-tert-butyl-2-méthyl-1-phényl)propanal ;
b. 5-tert-butyl-2-indanecarbaldéhyde ;
c. 5-tert-butyl-2-méthyl-2-indanecarbaldéhyde ;
d. 3-(3-tert-butyl-5-méthylphényl)propanal ;
e. 6-tert-butyl-1-indaneacétaldéhyde ;
f. 2-(5-tert-butyl-2-indanyl)-1,3-dioxolane ;
g. 3-(3,3-diméthyl-5-indanyl)propanal ;
h. 3-(1,1-diméthyl-5-indanyl)propanal ;
i. 3-(5-indanyl)propanal ; et
j. 3-(4-indanyl)propanal.

**3.** Utilisation d'un composé selon la revendication 1 ou 2 à titre d'ingrédient parfumant.

**4.** Composition parfumante ou article parfumé contenant en tant qu'ingrédient actif un composé selon la revendication 1 ou 2.

**5.** Article parfumé selon la revendication 4, sous forme d'un parfum ou d'une eau de toilette, d'un savon, d'un gel de douche ou bain, d'un shampoing ou d'un produit après-shampoing, d'une préparation cosmétique, d'un désodorisant corporel ou d'air ambiant, d'un détergent ou adoucissant textile ou d'un produit d'entretien.

**6.** Procédé pour la préparation d'un composé de formule

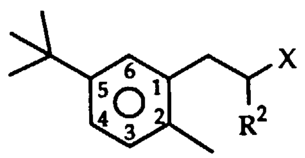

(I'a)

dans laquelle les symboles X et $R^2$ ont le sens indiqué à la formule (Ia), ou de formule

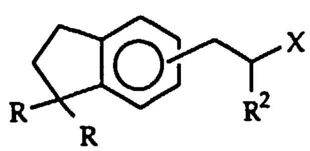

(Ic)

telle que définie à la revendication 1, le procédé étant caractérisé en ce qu'on hydrolyse, à l'aide d'un acide, un énol-ester de formule

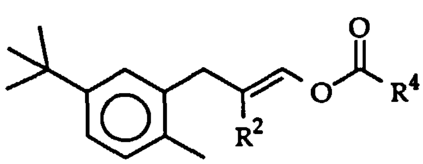

(IIa)

ou, respectivement, de formule

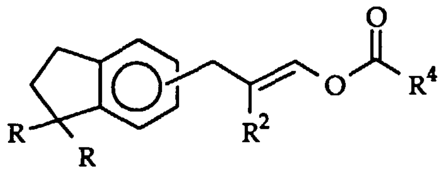

(IIc)

dans lesquelles les symboles R et $R^2$ ont le sens indiqué ci-dessus et le symbole $R^4$ représente un radical alkyle de $C_1$ à $C_3$ et, le cas échéant, on acétalyse, de façon connue en soi, l'aldéhyde (I'a), respectivement (Ic), ainsi obtenu pour former l'acétal correspondant.

**7.** Enol-ester de formule

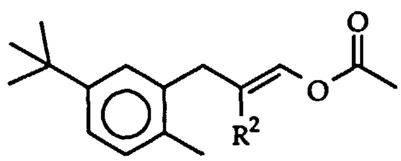

ou de formule

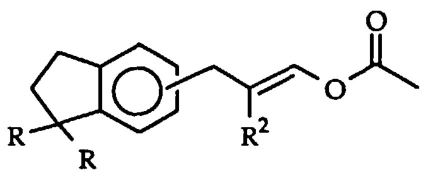

dans laquelle R et $R^2$ représentent un atome d'hydrogène ou un radical méthyle et les groupes R sont identiques ou différents.

**8.** Procédé pour la préparation d'un composé de formule

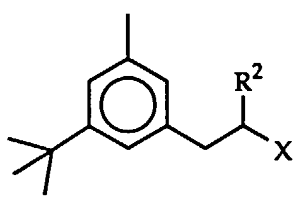

(I"a)

dans laquelle X et $R^2$ sont définis comme à la formule (Ia), caractérisé en ce qu'on soumet à une hydrogénation catalytique, dans un solvant organique inerte, un aldéhyde de formule

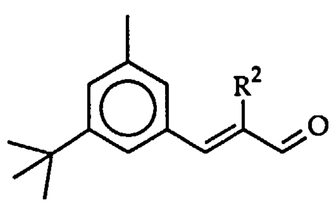

(III)

dans laquelle $R^2$ a le sens indiqué ci-dessus et, le cas échéant, on acétalyse, de façon connue en soi, l'aldéhyde (I"a) ainsi obtenu pour former l'acétal correspondant.

**9.** Procédé selon la revendication 7, caractérisé en ce qu'on utilise en tant que produit de départ le 3-(3-tert-butyl-5-méthyl-1-phényl)-2-propénal, ce dernier étant obtenu par réaction de 4-tert-butyl-1-(diméthoxyméthyl)-2-méthylènebenzène avec l'éthyl vinyl éther, en présence de chlorure de zinc et d'acide phosphorique.

**10.** Composé de formule

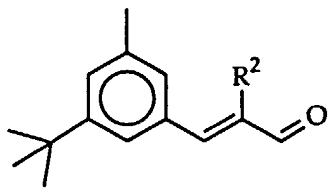

(III)

dans laquelle $R^2$ représente un atome d'hydrogène ou un radical méthyle.

**11.** Procédé pour la préparation d'un composé de formule

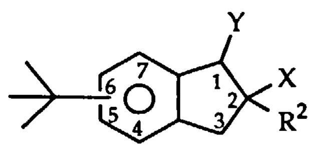

(Ib)

dans laquelle le radical tert-butyle se trouve en position 5 ou 6 du cycle aromatique et soit Y représente l'hydrogène et X et $R^2$ sont définis comme à la formule (Ia), soit X et $R^2$ représentent l'hydrogène et Y représente un groupe - $CH_2CHO$ ou un groupe

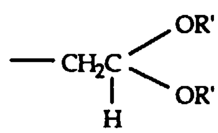

R' ayant le sens indiqué à la formule (Ia), le procédé étant caractérisé en ce qu'on

a. traite avec un agent oxydant un alcool de formule

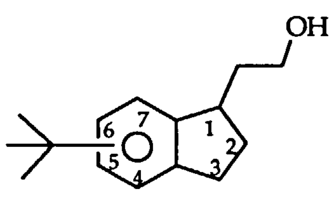

(IVa)

ou

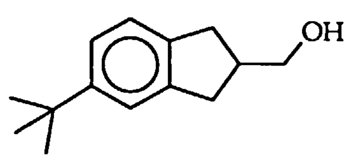

(IVb)

pour former l'aldéhyde (Ib) correspondant ;
b. le cas échéant, on soumet l'aldéhyde obtenu en a., correspondant à l'alcool (IVb), à une méthylation, de façon connue en soi, pour former l'aldéhyde (Ib) de formule

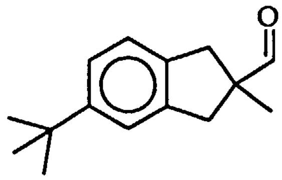

;

et

c. le cas échéant, on acétalyse, de façon connue en soi, l'aldéhyde (Ib) pour former l'acétal correspondant.

**12.** Composé de formule

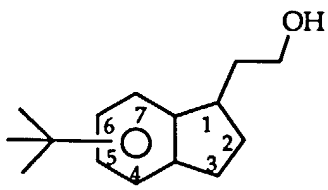

(IVa)

dans laquelle le radical tert-butyle se trouve en position 5 ou 6 du cycle aromatique, ou de formule

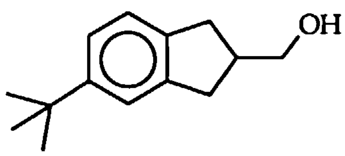

(IVb)

## Patentansprüche

**1.** Verbindung mit der Formel

a)

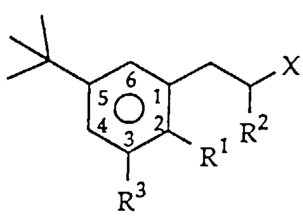

(Ia),

in der das Symbol X für eine Gruppe -CHO oder eine Gruppe

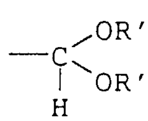

steht, wobei die Symbole R' für sich genommen jeweils für einen linearen oder verzweigten, gesättigten

oder ungesättigten $C_1$-$C_4$-Alkylrest oder insgesamt für einen gegebenenfalls substituierten $C_2$-$C_4$-Alkylenrest stehen; das Symbol $R^2$ für ein Wasserstoffatom oder einen Methylrest steht; und $R^1$ und $R^3$ verschieden sind und jeweils für ein Wasserstoffatom oder einen Methylrest stehen ; mit Ausnahme von 2-(5-tert-Butyl-2-methylbenzyl)propanal ;
oder mit der Formel
b)

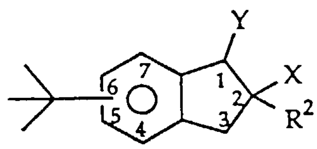

(Ib),

in der sich der tert-Butylrest in der Position 5 oder 6 des aromatischen Ringes befindet, und entweder Y für Wasserstoff steht und X und $R^2$ die oben angegebene Bedeutung haben, oder X und $R^2$ jeweils für ein Wasserstoffatom stehen und Y für eine Gruppe $-CH_2CHO$ oder eine Gruppe

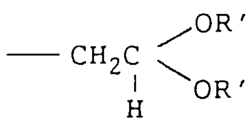

steht, wobei R' wie unter a) definiert ist;
oder mit der Formel
c)

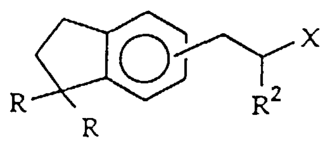

(Ic),

in der X und $R^2$ die in Formel (Ia) angegebene Bedeutung haben und R für ein Wasserstoffatom oder einen Methylrest steht, wobei die R-Gruppen identisch oder verschieden sein können.

**2.** Als Verbindung gemäß Anspruch 1, eine der folgenden Verbindungen:

a. 3-(5-tert-Butyl-2-methyl-1-phenyl)propanal;
b. 5-tert-Butyl-2-indancarbaldehyd;
c. 5-tert-Butyl-2-methyl-2-indancarbaldehyd;
d. 3-(3-tert-Butyl-5-methylphenyl)propanal;
e. 6-tert-Butyl-1-indanacetaldehyd;
f. 2-(5-tert-Butyl-2-indanyl)-1,3-dioxolan;
g. 3-(3,3-Dimethyl-5-indanyl)propanal;
h. 3-(1,1-Dimethyl-5-indanyl)propanal;
i. 3-(5-Indanyl)propanal; und
j. 3-(4-Indanyl)propanal.

**3.** Verwendung einer Verbindung gemäß Anspruch 1 oder 2 als Riechstoff.

**4.** Duftzusammensetzung oder parfümierter Artikel, welche bzw. welcher als aktiven Inhaltsstoff eine Verbindung gemäß Anspruch 1 oder 2 enthält.

**5.** Parfümierter Artikel gemäß Anspruch 4 in Form eines Parfüms oder Eau de Toilette, einer Seife, eines Dusch-

oder Badegels, eines Shampoo oder eines Produktes zur Verwendung nach der Haarwäsche, eines kosmetischen Präparats, eines Körper- oder Raumluftdeodorants, eines Detergens oder Textilweichspülers oder eines Universalreinigers.

**6.** Verfahren zur Herstellung einer Verbindung mit der Formel

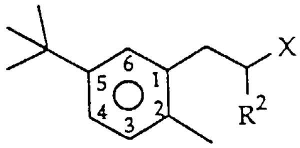

(I'a),

in der die Symbole X und $R^2$ die in Formel (Ia) angegebene Bedeutung haben, oder mit der Formel

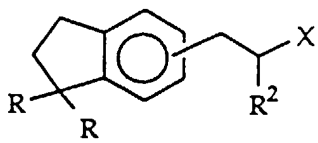

(Ic)

gemäß der Definition in Anspruch 1, wobei das Verfahren dadurch gekennzeichnet ist, daß mit Hilfe einer Säure ein Enolester mit der Formel

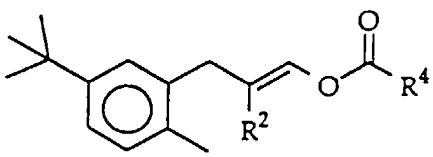

(IIa)

bzw. mit der Formel

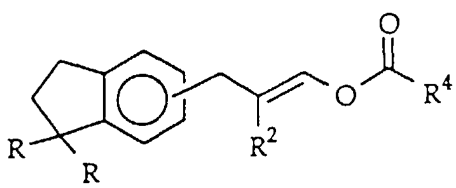

(IIc)

hydrolysiert wird, in welchen die Symbole R und $R^2$ die oben angegebene Bedeutung haben und das Symbol $R^4$ für einen $C_1$-$C_3$-Alkylrest steht, und der auf diese Weise erhaltene Aldehyd (I'a) bzw. (Ic) gegebenenfalls auf an sich bekannte Weise acetalisiert wird, um das entsprechende Acetal zu bilden.

**7.** Enolester mit der Formel

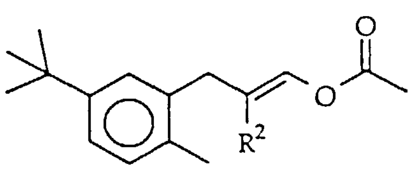

oder mit der Formel

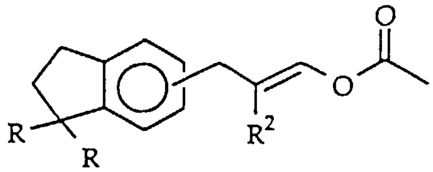

,

in der R und $R^2$ für ein Wasserstoffatom oder einen Methylrest stehen und die R-Gruppen identisch oder verschieden sind.

**8.** Verfahren zur Herstellung einer Verbindung mit der Formel

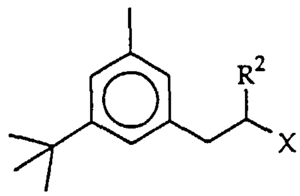

(I"a),

in der X und $R^2$ wie in der Formel (Ia) definiert sind, dadurch gekennzeichnet, daß ein Aldehyd mit der Formel

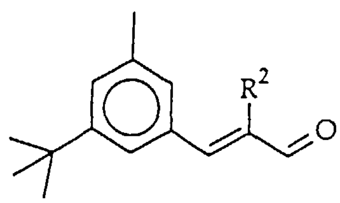

(III),

in der $R^2$ die oben angegebene Bedeutung besitzt, einer katalytischen Hydrierung in einem inerten organischen Lösungsmittel unterzogen wird, und der auf diese Weise erhaltene Aldehyd (I"a) gegebenenfalls auf an sich bekannte Weise acetalisiert wird, um das entsprechende Acetal zu bilden.

**9.** Verfahren gemäß Anspruch 7, dadurch gekennzeichnet, daß als Ausgangsprodukt 3-(3-tert-Butyl-5-methyl-1-phenyl)-2-propenal eingesetzt wird, wobei dieses mittels Umsetzung von 4-tert-Butyl-1-(dimethoxymethyl)-2-methylenbenzol mit Ethylvinylether in Gegenwart von Zinkchlorid und Phosphorsäure hergestellt wird.

**10.** Verbindung mit der Formel

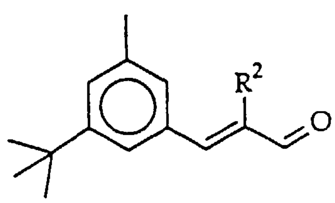

(III),

in der $R^2$ für ein Wasserstoffatom oder einen Methylrest steht.

**11.** Verfahren zur Herstellung einer Verbindung mit der Formel

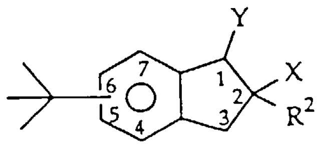

(Ib),

in der sich der tert-Butylrest in der Position 5 oder 6 des aromatischen Rings befindet, und entweder Y für Wasserstoff steht und X und $R^2$ wie in der Formel (Ia) definiert sind, oder X und $R^2$ für Wasserstoff stehen und Y für eine Gruppe $-CH_2CHO$ oder eine Gruppe

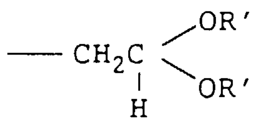

steht,
und R' die in Formel (Ia) angegebene Bedeutung hat, wobei das Verfahren dadurch gekennzeichnet ist, daß

a. ein Alkohol mit der Formel

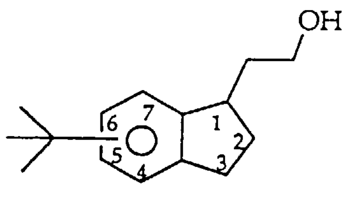

oder

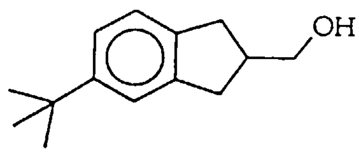

(IVa) (IVb)

mit einem Oxidationsmittel behandelt wird, um den entsprechenden Aldehyd (Ib) zu bilden;
b. gegebenenfalls der unter a. hergestellte Aldehyd, der dem Alkohol (IVb) entspricht, auf an sich bekannte Weise einer Methylierung unterzogen wird, um den Aldehyd (Ib) mit der Formel

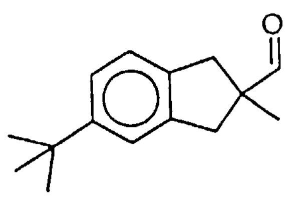

zu bilden;
und
c. gegebenenfalls der Aldehyd (Ib) auf an sich bekannte Weise acetalisiert wird, um das entsprechende Acetal zu bilden.

**12.** Verbindung mit der Formel

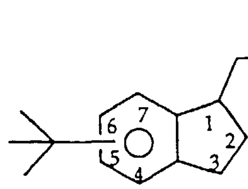

(IVa),

in der sich der tert-Butylrest in der Position 5 oder 6 des aromatischen Ringes befindet, oder mit der Formel

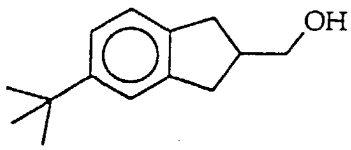

(IVb).

**Claims**

**1.** A compound of formula

a.

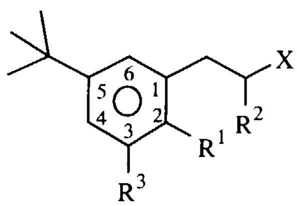

(Ia)

wherein symbol X represents a - CHO group or a group

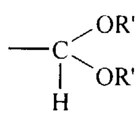

in which symbols R', taken separately, represent each a $C_1$ to $C_4$, linear or branched, saturated or unsaturated alkyl radical, or taken together represent a substituted or unsubstituted $C_2$ to $C_4$ alkylene radical ; symbol $R^2$ represents a hydrogen atom or a methyl radical ; and $R^1$ and $R^3$ are different and represent each a hydrogen atom or a methyl radical, provided that 2-(5-tert-butyl-2-methylbenzyl)propanal is excluded ;
or of formula

b.

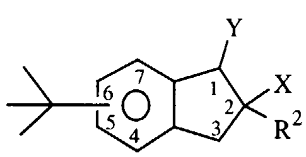

(Ib)

wherein the tert-butyl radical is located in position 5 or 6 of the aromatic ring and, either Y represents hydrogen and X and $R^2$ have the meaning indicated above, or X and $R^2$ represent each a hydrogen atom and

Y represents a $-CH_2CHO$ group or a group

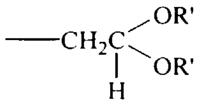

in which R' is defined as in a. ;
or of formula
c.

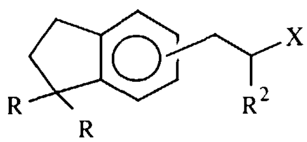

(Ic)

wherein X and $R^2$ have the meaning indicated in formula (Ia) and R represents a hydrogen atom or a methyl radical, the groups R being identical or different.

**2.** As a compound according to claim 1, one of the following compounds :

a. 3-(5-tert-butyl-2-methyl-1-phenyl)propanal ;
b. 5-tert-butyl-2-indancarbaldehyde ;
c. 5-tert-butyl-2-methyl-2-indancarbaldehyde ;
d. 3-(3-tert-butyl-5-methylphenyl)propanal ;
e. 6-tert-butyl-1-indanacetaldehyde ;
f. 2-(5-tert-butyl-2-indanyl)-1,3-dioxolane ;
g. 3-(3,3-dimethyl-5-indanyl)propanal ;
h. 3-(1,1-dimdthyl-5-indanyl)propanal ;
i. 3-(5-indanyl)propanal ; and
j. 3-(4-indanyl)propanal.

**3.** Use of a compound according to claim 1 or 2, as a perfuming ingredient.

**4.** A perfuming composition or a perfumed article which contains as an active ingredient a compound according to claim 1 or 2.

**5.** A perfumed article according to claim 4, in the form of a perfume, a cologne, a soap, a bath or shower gel, a shampoo or other hair-care product, a cosmetic preparation, a body deodorant or an air-freshener, a detergent or a fabric softener, or a household product.

**6.** A process for the preparation of a compound of formula

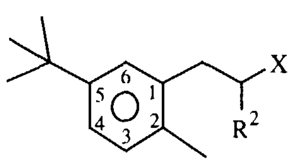

(I'a)

wherein symbols X and $R^2$ have the meaning indicated in formula (Ia), or of formula

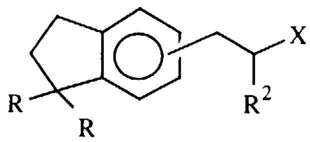

(Ic)

such as defined in claim 1, characterized in that an enol-ester of formula

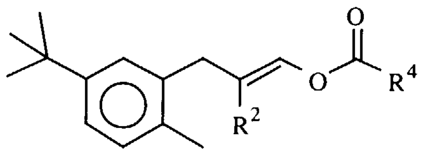

(IIa)

or respectively of formula

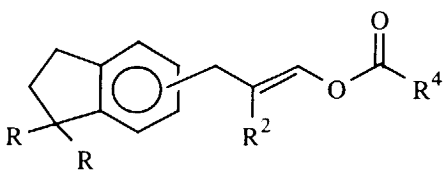

(IIc)

in which formulae R and $R^2$ have the meaning indicated above and symbol $R^4$ represents a $C_1$ to $C_3$ alkyl radical, is hydrolyzed by means of an acid, and, where applicable, the aldehyde (I'a), respectively (Ic) thus formed is acetalyzed, in a generally known manner, to provide the corresponding acetal.

**7.** An enol-ester of formula

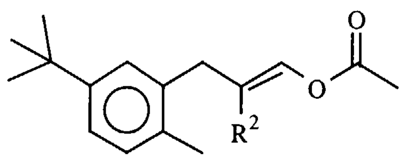

or of formula

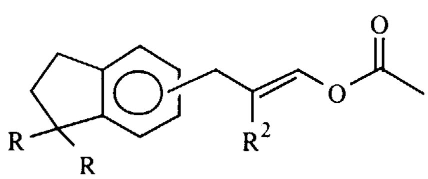

wherein R and $R^2$ represent a hydrogen atom or a methyl radical and the groups R can be identical or different.

**8.** A process for the preparation of a compound of formula

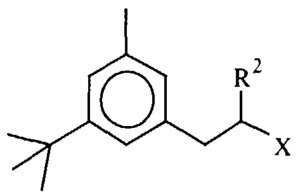

(I"a)

wherein X and $R^2$ are defined as in formula (Ia), characterized in that an aldehyde of formula

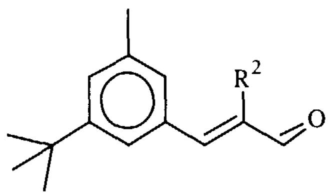

(III)

wherein $R^2$ has the meaning indicated above, is subjected to catalytic hydrogenation, in an inert organic solvent and, where applicable, the aldehyde (I"a) thus formed is acetalyzed, in a generally known manner, to provide the corresponding acetal.

**9.** The process of claim 7, characterized in that 3-(3-tert-butyl-5-methyl-1-phenyl)-2-propenal is used as starting product, the latter being obtained by reacting 4-tert-butyl-1-(dimethoxymethyl)-2-methylenebenzene with ethyl vinyl ether, in the presence of zinc chloride and phosphoric acid.

**10.** A compound of formula

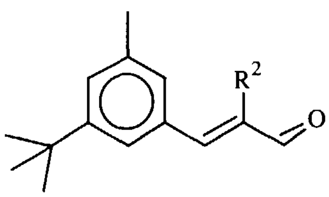

(III)

wherein $R^2$ represents a hydrogen atom or a methyl radical.

**11.** A process for the preparation of a compound of formula

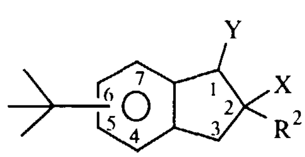

(Ib)

wherein the tert-butyl radical is located in position 5 or 6 of the aromatic ring and either Y represents hydrogen and X and $R^2$ are defined as in formula (Ia), or X and $R^2$ represent hydrogen and Y represents a - $CH_2CHO$ or a group of formula

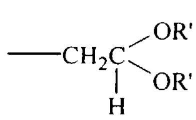

R' having the meaning indicated in formula (Ia), which process is characterized in that:

a. an alcohol of formula

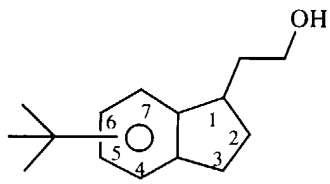

(IVa)

or

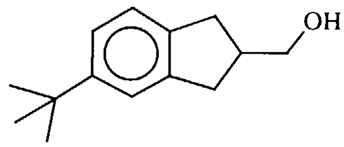

(IVb)

is treated with an oxidizing agent to form the corresponding aldehyde (Ib) ;

b. where applicable, the aldehyde obtained in a. which corresponds to alcohol (IVb) is methylated, in a generally known manner, to form an aldehyde (Ib) of formula

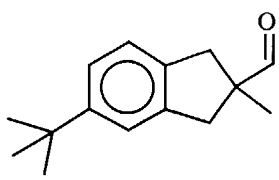

;

and

c. where applicable, said aldehyde (Ib) is acetalized, in a generally known manner, to form the corresponding acetal.

**12.** A compound of formula

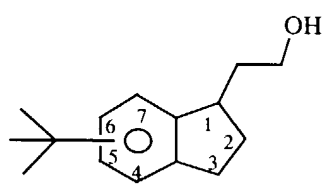

(IVa)

wherein the tert-butyl radical is located in position 5 or 6 of the aromatic ring,
or of formula

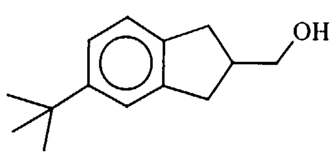

(IVb)

Figure 1

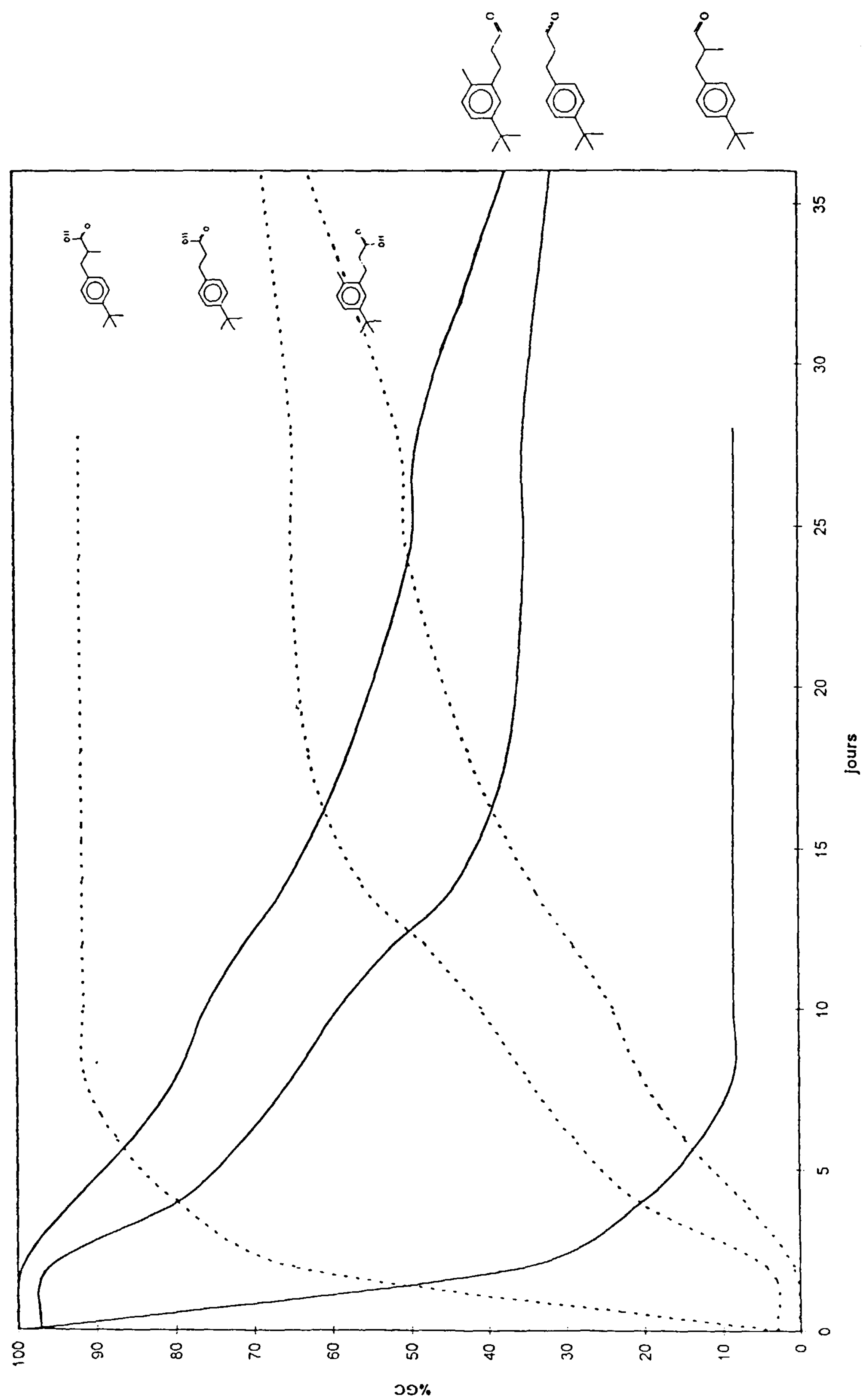

# Figure 2